# EUROPÄISCHE PATENTSCHRIFT

(11) **EP 3 478 739 B1**
(45) Veröffentlichungstag und Bekanntmachung des Hinweises auf die Patenterteilung: **25.06.2025**
(21) Anmeldenummer: 17734088.2
(22) Anmeldetag: 29.06.2017
(51) Int. Cl.: C08G 18/08, C08G 18/48, C08G 18/73, C08G 18/75, C08G 18/10, C08G 18/38, C09J 175/02, C09J 175/08, C08G 18/72, C08G 18/79, C08G 18/12, C09J 175/12, A61L 24/04, A61L 15/58, A61L 15/26

(54) **KONTAKTKLEBENDES PRODUKT BASIEREND AUF POLYURETHANHARNSTOFF, DESSEN HERSTELLUNG SOWIE DER ENTSPRECHENDE KONTAKTKLEBSTOFF**
CONTACT ADHESIVE PRODUCT BASED ON POLYURETHANE UREA, THE PRODUCTION THEREOF AND CORRESPONDING CONTACT ADHESIVE
PRODUIT D'ADHERENCE DE CONTACT A BASE D'UREE DE POLYURETHANE, SA FABRICATION ET PRODUIT D'ADHERENCE DE CONTACT

(30) Priorität: 30.06.2016 EP 16177190
(43) Veröffentlichungstag der Anmeldung: 08.05.2019
(73) Patentinhaber: Covestro Deutschland AG, 51373 Leverkusen (DE)
(72) Erfinder: DÖRR, Sebastian, 40593 Düsseldorf (DE); WEISER, Marc-Stephan, 51515 Kürten-Dürscheid (DE); PLUG, Sascha, 51375 Leverkusen (DE)
(74) Vertreter: Levpat
(86) Internationale Anmeldenummer: PCT/EP2017/066197
(87) Internationale Veröffentlichungsnummer: WO 2018/002257

(56) Entgegenhaltungen:
- EP-A1- 2 332 998
- WO-A1-01/62818
- WO-A1-2010/142393
- WO-A1-2013/136108

## Beschreibung

Die vorliegende Erfindung betrifft ein kontaktklebendes Produkt, umfassend ein Substrat und einen speziellen Polyurethanharnstoff, sowie ein Verfahren zur Herstellung des kontaktklebenden Produktes. Ebenfalls Gegenstand der Erfindung sind ein spezieller Polyurethanharnstoff und ein Kontaktklebstoff umfassend diesen Polyurethanharnstoff.

In vielen, insbesondere medizinischen Anwendungen, wie beispielsweise Bandagen für die Kompressionstherapie kommen Kontaktklebstoffe zum Einsatz. Wichtig ist dabei, dass Klebstoffe verwendet werden, die es ermöglichen die Produkte unter anderem auf Rollen zu lagern, ohne das die einzelnen Schichten so stark verkleben, dass sie nicht mehr oder nur unter erheblichem Kraftaufwand von der Rolle abgewickelt werden können. Gleichzeitig sollen die Schichten jedoch bei der Anwendung am menschlichen Körper nur durch leichten Druck oder Zugspannung, jedoch ohne Einwirkung von Wärme, Strahlung oder ähnlichen äußeren Effekten gut aufeinander haften und die Bandage zuverlässig verschließen, dabei jedoch nicht auf Haut, Haaren oder Kleidung kleben und auch reversibel ablösbar und im Idealfall sogar bei gleichbleibenden Klebeeigenschaften mehrfach wiederverwendbar sein.

Oft werden in solchen Produkten Latexformulierungen auf Basis von Naturkautschuken als klebende Komponenten eingesetzt. Dies bringt jedoch Nachteile wie Verfärbung bei Alterung, oft einen unangenehmen Geruch und auch nicht selten allergische Reaktionen der Haut mit sich.

So beschreibt unter anderem die US 6,156,424 kontaktklebende Produkte zur Verwendung in Bandagen oder Verbänden basierend auf mit wasserbasierten Polymeren getränkten Substraten. Die verwendeten Polymere sind inhärent kristalline elastomere Polymere wie Polychloroprene, aber auch Polyesterpolyurethane oder Polycaprolactonurethane. Nachteilig ist dabei, dass diese nur in Kombination mit bestimmten Klebrigmachern (Tackifiern) einsetzbar sind. Es muss stets eine vollständige Kristallisation der Produkte vermieden werden, da diese sonst ihre Klebkraft verlieren. Dies ist insbesondere bei langer Lagerung schwer zu gewährleisten.

Die US 5,692,937 beschreibt Kontaktklebstoffe, die für dehnbare Produkte wie Bandagen geeignet sind und auf wässrigen Dispersionen von Polyesterpolyurethanen basieren. Die dort beschriebenen Produkte weisen jedoch eine geringe Kontaktklebrigkeit auf, die für viele Anwendungen alleine nicht ausreichend ist und werden daher bevorzugt in Kombination mit anderen Klebstoffdispersionen basierend auf eher unerwünschten Polyacrylaten eingesetzt. Darüber hinaus weisen die Dispersionen sowie die daraus gebildeten Klebefilme eine gelbbraune Farbe auf, die als Kontaktklebstoff für medizinische Produkte eher ungeeignet ist, da sie eine unhygienische und schmutzige Erscheinung des Produktes hervorrufen.

Auch die CN104725589 A beschreibt bereits die Verwendung von wässrigen Polyurethan-dispersionen für selbst-klebende, elastische Bandagen. Die dort beschriebenen Bandagen weisen jedoch ebenfalls eine nicht ausreichende Kontaktklebrigkeit auf.

Eine Aufgabe der vorliegenden Erfindung war es mindestens einen Nachteil des Standes der Technik zumindest zu einem Teil zu überwinden.

In WO 2010/142393 A1 werden wässrige Polyurethandispersionen offenbart, welche durch Umsetzung von mindestens zwei Poly(tetramethylenglycol)polyetherpolyolen mit unterschiedlichen mittleren Molekularmassen und mindestens zwei unterschiedlichen Polyisocyanat-Komponenten hergestellt werden.

EP 2 332 998 A1 werden lösungsmittelfreie, wässrige Polyurethan-Dispersionen und Verfahren zur Herstellung und deren Verwendung beschrieben.

WO 01/62818 A1 offenbart Klebstoffe auf Basis von Polyurethanen, wobei diese Polyurethane das Reaktionsprodukt einer Isocyanat-reaktiven Komponente enthaltend mindestens zwei Isocyanat-reaktive Materialien und einer Isocyanat-funktionellen Komponente ist. Hierbei weist das erste Isocyanat-reaktives Material ein durchschnittliches Molekulargewicht von weniger als 2000 g/mol und das zweite von mindestens 2000 g/mol auf.

WO 2013/136108 bezieht sich auf die Verwendung einer Klebstoffzusammensetzung, die mindestens ein silylhaltiges Polymer, mindestens ein kompatibles Klebrigmacherharz und mindestens einen Katalysator umfasst, um einen atmungsaktiven selbstklebenden Gegenstand herzustellen. Dieses Stand der Technik Dokument betrifft auch eine Haftklebstoffzusammensetzung als auch einen atmungsaktiven selbstklebenden Gegenstand, der mindestens ein atmungsaktives Substrat umfasst, das mit einer atmungsaktiven Klebeschicht beschichtet ist. Eine weitere Aufgabe der vorliegenden Erfindung war es ein kontaktklebendes Produkt bereitzustellen, das zum einen auf Rollen gelagert werden kann, ohne das die einzelnen Schichten stark verkleben, zum anderen aber die Schichten bei der Anwendung am menschlichen Körper ohne Einwirkung von Wärme oder ähnlichem eine gute Verklebung untereinander aufweisen.

Weiterhin war es eine Aufgabe der vorliegenden Erfindung ein kontaktklebendes Produkt bereitzustellen, das eine hohe Farbechtheit, insbesondere eine hohe Lichtstabilität aufweist.

Mindestens eine dieser Aufgaben konnte überraschend gelöst werden durch die Bereitstellung eines kontaktklebenden Produkten, umfassend ein Substrat und einen Polyurethanharnstoff, welcher erhältlich ist, durch Umsetzung von wenigstens
A) einer aliphatischen Polyisocyanat-Komponente mit einer mittleren Isocyanatfunktionalität von ≥ 1,8 und ≤ 2,6,
B) einer polymeren Polyether-Polyol-Komponente, welche ein Gemisch aus Poly(tetramethylenglykol)polyetherpolyolen enthält oder daraus besteht, wobei sich die Poly(tetramethylenglykol)polyetherpolyole in ihren zahlenmittleren Molekulargewichten unterscheiden,
C) einer aminofunktionellen Kettenverlängerer-Komponente mit mindestens 2 isocyanatreaktiven Aminogruppen, enthaltend wenigstens eine aminofunktionelle Verbindung C1), die keine ionischen oder ionogenen Gruppen aufweist und eine aminofunktionelle Verbindung C2), die ionische oder ionogene Gruppen aufweist,
D) gegebenenfalls weiteren hydrophilierenden Komponenten, die unterschiedlich sind von C2),
E) gegebenenfalls hydroxyfunktionellen Verbindungen mit einem Molekulargewicht von 62 bis 399 mol/g,
F) gegebenenfalls weiteren polymeren Polyolen, welche unterschiedlich sind von B),
G) einer Verbindung, die genau eine isocyanatreaktive Gruppe aufweist, oder einer Verbindung, die mehr als eine isocyanatreaktive Gruppe aufweist, wobei nur eine der isocyanatreaktiven Gruppen unter den gewählten Umsetzungsbedingungen mit den in der Reaktionsmischung vorhandenen Isocyanatgruppen reagiert und
H) einer aliphatischen Polyisocyanat-Komponente mit einer mittleren Isocyanatfunktionalität von > 2,6 und ≤ 4,

wobei das molare Verhältnis von Komponente G) zu Komponente H) 5:1 bis 1:5 beträgt und
wobei die Komponenten B) und F) zusammen ≤ 30 Gew.-% an Komponente F), bezogen auf die Gesamtmasse der Komponenten B) und F) enthalten.

Es konnte überraschend gezeigt werden, dass basierend auf den genannten speziellen Polyurethanharnstoffen kontaktklebende Produkte erhalten werden konnten, die zum Einen auf Rollen gelagert werden können, ohne dass die einzelnen Schichten stark verkleben und zum Anderen aber die Schichten bei der Anwendung am menschlichen Körper ohne Einwirkung von Wärme oder ähnlichem eine gute Verklebung untereinander aufweisen.

Kontaktklebend im Sinne dieser Erfindung bedeutet, dass eine einzelne Schicht des betreffenden Materials (Kontaktklebstoff), bzw. das Produkt an sich keine oder nur eine sehr geringe Klebrigkeit aufweist. Erst bei Kontakt und bevorzugt Verpressen mit einer zweiten Schicht desselben Materials oder Produktes entsteht eine Verklebung der beiden Materialschichten mit einer guten Klebkraft. Der Kontaktklebstoff muss folglich auf beide zu verbindenden Teile oder Schichten aufgebracht werden und wird im Anschluss bevorzugt getrocknet bis keine fühlbare Klebrigkeit mehr auftritt.

Polyurethanharnstoffe im Sinne der Erfindung sind polymere Verbindungen, die mindestens zwei, bevorzugt mindestens drei Urethan-Gruppen-haltige Wiederholungseinheiten aufweisen:

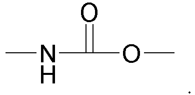

Erfindungsgemäß weisen die Polyurethanharnstoffe herstellungsbedingt auch HarnstoffGruppen-haltige Wiederholungseinheiten auf, 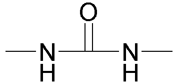 wie sie insbesondere bei der Umsetzung von isocyanatterminierten Präpolymeren mit aminofunktionellen Verbindungen gebildet werden.

Unter ionogenen Gruppen werden im Sinne dieser Erfindung solche funktionellen Gruppen verstanden, die in der Lage sind, beispielsweise durch Neutralisation mit einer Base, ionische Gruppen zu bilden.

Die Komponente A) kann jedes Polyisocyanat sein, das der Fachmann hierfür verwenden würde. Als Komponente A) bevorzugt geeignete Polyisocyanate sind insbesondere die dem Fachmann an sich bekannten aliphatischen Polyisocyanate mit einer mittleren Isocyanatfunktionalität von ≥ 1,8 und ≤ 2,6. Der Begriff aliphatisch umfasst dabei auch cycloaliphatische und/oder araliphatische Polyisocyanate.

Unter der mittleren Isocyanatfunktionalität wird dabei die durchschnittliche Anzahl an Isocyanatgruppen pro Molekül verstanden.

Bevorzugte Polyisocyanate sind solche des Molekulargewichtsbereichs von 140 bis 336 g/mol. Besonders bevorzugt sind diese ausgewählt aus der Gruppe bestehend aus. 1,4-Diisocyanatobutan (BDI), 1,5-Pentandiisocyanat, (PDI) 1,6-Diisocyanatohexan (HDI), 1,3-Bis(isocyanatomethyl)benzol (1,3-Xylylendiisocyanat, XDI), 1,4-Bis(isocyanatomethyl)benzol (1,4-Xylylendiisocyanat, XDI), 1,3-Bis(1-isocyanato-1-methyl-ethyl)benzol (TMXDI), 1,4-Bis(1-isocyanato-1-methyl-ethyl)benzol (TMXDI), 4-Isocyanatomethyl-1,8-octandiisocyanat (Trisisocyanatononan (TIN)), 2-Methyl-1,5-diisocyanatopentan, 1,5-Diisocyanato-2,2-dimethylpentan, 2,2,4- bzw. 2,4,4-Trimethyl-1,6-diisocyanatohexan, 1,10-Diisocyanatodecan sowie die cycloaliphatischen Diisocyanate 1,3-bzw. 1,4-Diisocyanatocyclohexan, 1,4-Diisocyanato-3,3,5-trimethylcyclohexan, 1,3-Diisocyanato-2(4)-methylcyclohexan, 1-Isocyanato-3,3,5-trimethyl-5-isocyanatomethylcyclohexan (Isophorondiisocyanat, IPDI), 1-Isocyanato-1-methyl-4(3)isocyanato-methylcyclohexan, 1,8-Diisocyanato-p-menthan, 4,4'-Diisocyanato-1,1'-bi(cyclohexyl), 4,4'-Diisocyanato-3,3'-dimethyl-1,1'-bi(cyclohexyl), 4,4'-Diisocyanato-2,2',5,5'-tetramethyl-1,1'-bi(cyclohexyl), 4,4'- und/oder 2,4'-Diisocyanatodicyclohexylmethan, 4,4'-Diisocyanato-3,3'-dimethyldicyclohexylmethan, 4,4'-Diisocyanato-3,3',5,5'-tetramethyldicyclohexylmethan, 1,3-Diisocyanatoadamantan, und 1,3-Dimethyl-5,7-diisocyanatoadamantan oder beliebige Gemische solcher Isocyanate. Ganz besonders bevorzugt sind die Polyisocyanate ausgewählt aus 1,4-Butylendiisocyanat, 1,5-Pentamethylendiisocyanat (PDI), 1,6-Hexamethylendiisocyanat (HDI), Isophorondiisocyanat (IPDI), 2,2,4- und/oder 2,4,4-Trimethylhexamethylendiisocyanat, die isomeren Bis-(4,4'-isocyanatocyclohexyl)methane oder deren Mischungen beliebigen Isomerengehalts (H12-MDI), 1,4-Cyclohexylendiisocyanat, 4-Isocyanatomethyl-1,8-octandiisocyanat (Nonantriisocyanat) sowie Alkyl-2,6-diisocyanatohexanoate (Lysindiisocyanate) mit C1-C8-Alkylgruppen.

Neben den vorstehend genannten Polyisocyanaten können auch modifizierte Diisocyanate, die eine mittlere Isocyanatfunktionalität ≥ 2 und ≤ 2,6 aufweisen, mit Uretdion-, Isocyanurat-, Urethan-, Allophanat-, Biuret-, Iminooxadiazindion- oder Oxadiazintrionstruktur sowie Mischungen aus diesen und/oder den oben stehenden anteilig eingesetzt werden.

Bevorzugt handelt es sich um Polyisocyanate oder Polyisocyanatgemische der vorstehend genannten Art mit ausschließlich aliphatisch oder cycloaliphatisch gebundenen Isocyanatgruppen oder Mischungen aus diesen und einer mittleren NCO-Funktionalität der Mischung von ≥ 1,8 und ≤ 2,6 und besonders bevorzugt ≥ 2,0 und ≤ 2,4.

Besonders bevorzugt enthält die organische Polyisocyanat-Komponente A) ein aliphatisches oder cycloaliphatisches Polyisocyanat ausgewählt aus HDI, IPDI und/oder H12-MDI oder deren Modifikationsprodukten, ganz besonders bevorzugt ausgewählt aus HDI und/oder IPDI.

In einer insbesondere bevorzugten Variante liegen als Komponente A) IPDI und HDI im Gemisch vor.

Das Gewichtsverhältnis von IPDI:HDI liegt dabei bevorzugt im Bereich von 1,05 bis 10, besonders bevorzugt im Bereich von 1,1 bis 5, und ganz besonders bevorzugt im Bereich von 1,1 bis 1,5.

In einer bevorzugten Ausführungsform wird zur Herstellung des erfindungsgemäß verwendeten Polyurethanharnstoffs ≥ 5 und ≤ 40 Gew.-% der Komponente A) und besonders bevorzugt ≥ 10 und ≤ 35 Gew.-% der Komponente A), jeweils bezogen auf die Gesamtmasse des Polyurethanharnstoffs, eingesetzt.

In einer weiteren bevorzugten Ausführungsform wird zur Herstellung des Polyurethanharnstoffs auch die Komponente H), eine aliphatische Polyisocyanat-Komponente mit einer mittleren Isocyanatfunktionalität (mittlere Anzahl Isocyanatgruppen pro Molekül) von > 2,6 und ≤ 4, vorzugsweise ≥ 2,8 und ≤ 3,8 eingesetzt. Die Komponente H) wird dabei bevorzugt im Gemisch mit Komponente A) eingesetzt.

Als Komponente H) besonders geeignet sind oligomere Diisocyanate, die eine Funktionalität > 2,6 und ≤ 4, vorzugsweise ≥ 2,8 und ≤ 3,8 aufweisen, mit Isocyanurat-, Urethan-, Allophanat-, Biuret-, Iminooxadiazindion- oder Oxadiazintrionstruktur. Ganz besonders bevorzugt enthält H) Isocyanuratstrukturen.

Besonders bevorzugt besteht die organische Polyisocyanat-Komponente H) aus einem aliphatischen oder cycloaliphatischen Polyisocyanat-Oligomer basierend auf HDI, IPDI und/oder H12-MDI, ganz besonders bevorzugt basierend auf HDI.

Das Molverhältnis der NCO-Gruppen aus Komponente A) zu Komponente H) beträgt dabei bevorzugt 100:0,5 bis 100: 50; besonders bevorzugt 100: 2 bis 100: 15 und ganz besonders bevorzugt 100:3 bis 100: 8.

In einer bevorzugten Ausführungsform wird zur Herstellung des erfindungsgemäß verwendeten Polyurethanharnstoffs ≥ 0 und ≤ 10 Gew.-% der Komponente H) und besonders bevorzugt ≥ 0,1 und ≤ 3 Gew.-% der Komponente H), jeweils bezogen auf die Gesamtmasse des Polyurethanharnstoffs, eingesetzt.

Die erfindungsgemäß als Komponente B) eingesetzten polymeren Polyether-Polyole weisen bevorzugt zahlenmittlere Molekulargewichte von ≥ 500 und ≤ 8000 g/mol, bestimmt durch Gelpermeationschromatographie gegenüber Polystyrol-Standard in Tetrahydrofuran bei 23°C, bevorzugter ≥ 400 und ≤ 6000 g/mol und besonders bevorzugt von ≥ 600 und ≤ 3000 g/mol und/oder OH-Funktionalitäten von bevorzugt ≥ 1,5 und ≤ 6, bevorzugter ≥ 1,8 und ≤ 3, besonders bevorzugt von ≥ 1,9 und ≤ 2,1 auf.

Der Ausdruck "polymere" Polyether-Polyole bedeutet hier insbesondere, dass die genannten Polyole mindestens drei, bevorzugter mindestens vier miteinander verbundene Wiederholungseinheiten aufweisen.

Das zahlenmittlere Molekulargewicht wird im Rahmen dieser Anmeldung bestimmt durch Gelpermeationschromatographie (GPC) in Tetrahydrofuran bei 23°C, außer wenn dies anders beschrieben wird. Es wird dabei vorgegangen nach DIN 55672-1: "Gelpermeationschromatographie, Teil 1 - Tetrahydrofuran als Elutionsmittel" (SECurity GPC-System von PSS Polymer Service, Flussrate 1,0 ml/min; Säulen: 2×PSS SDV linear M, 8×300 mm, 5 µm; RID-Detektor). Dabei werden Polystyrolproben bekannter Molmasse zur Kalibrierung verwendet. Die Berechnung des zahlenmittleren Molekulargewichts erfolgt softwaregestützt. Basislinienpunkte und Auswertegrenzen werden entsprechend der DIN 55672 Teil 1 festgelegt.

Geeignete Polyetherpolyole sind beispielsweise die an sich bekannten Additionsprodukte von Styroloxid, Ethylenoxid, Propylenoxid, Butylenoxid und/oder Epichlorhydrin an di- oder polyfunktionelle Startermoleküle. So sind insbesondere Polyalkylenglykole, wie Polyethylen-, Polypropylen- und/oder Polybutylenglykole anwendbar, insbesondere mit den oben genannten bevorzugten Molekulargewichten. Als geeignete Startermoleküle können alle dem Stand der Technik nach bekannten Verbindungen eingesetzt werden, wie zum Beispiel Wasser, Butyldiglykol, Glycerin, Diethylenglykol, Trimethyolpropan, Propylenglykol, Sorbit, Ethylendiamin, Triethanolamin, 1,4-Butandiol.

Geeignete Poly(tetramethylenglykol)polyetherpolyole sind beispielsweise durch Polymerisation von Tetrahydrofuran mittels kationischer Ringöffnung erhältlich sind.

Erfindungsgemäß enthält die Komponente B) ein Gemisch aus Poly(tetramethylenglykol)polyetherpolyolen oder besteht daraus, wobei sich die Poly(tetramethylenglykol)polyetherpolyole in ihren zahlenmittleren Molekulargewichten unterscheiden.

In einer besonders bevorzugten Ausführungsform enthält die Komponente B) ein Gemisch aus Poly(tetramethylenglykol)polyetherpolyolen I mit einem zahlenmittleren Molekulargewichte Mₙ in einem Bereich von ≥ 400 und ≤ 1500 g/mol, besonders bevorzugt in einem Bereich von ≥ 600 und ≤ 1200 g/mol, ganz besonders bevorzugt in einem Bereich von 1000 g/mol und Poly(tetramethylenglykol)polyetherpolyole II mit einem zahlenmittleren Molekulargewichte Mₙ in einem Bereich von ≥ 1500 und ≤ 8000 g/mol, besonders bevorzugt in einem Bereich von ≥ 1800 und ≤ 3000 g/mol, ganz besonders bevorzugt von 2000 g/mol.

Das Gewichtsverhältnis der Poly(tetramethylenglykol)polyetherpolyole I zu den Poly(tetramethylenglykol)polyetherpolyolen II liegt bevorzugt im Bereich von 0,1 bis 10, besonders bevorzugt im Bereich von 0,2 bis 10, ganz besonders bevorzugt im Bereich von 1 bis 6.

Erfindungsgemäß wird zur Herstellung des Polyurethanharnstoffs eine aminofunktionelle Kettenverlängerer-Komponente C) mit mindestens 2 isocyanatreaktiven Aminogruppen, enthaltend wenigstens eine aminofunktionelle Verbindung C1), die keine ionischen oder ionogenen Gruppen aufweist und eine aminofunktionelle Verbindung C2), die ionische oder ionogene Gruppen aufweist, eingesetzt.

Die aminofunktionellen Verbindungen der Komponente C) Komponente werden bevorzugt aus primären und/oder sekundären Diaminen ausgewählt. Insbesondere umfassen die aminofunktionellen Verbindungen C) mindestens ein Diamin.

In einer bevorzugten Ausführungsform des Produktes umfasst die aminofunktionelle Komponente C) wenigstens eine aminofunktionelle Verbindung C2), die ionische und/oder ionogene Gruppen aufweist.

In einer weiteren bevorzugten Ausführungsform der Erfindung umfasst die aminofunktionelle Komponente C) sowohl aminofunktionelle Verbindungen C2), die ionische und/oder ionogene Gruppe aufweisen, als auch aminofunktionelle Verbindungen C1), die keine ionische oder ionogene Gruppe aufweisen.

Beispielsweise können als Komponente C1) organische Di- oder Polyamine wie beispielsweise 1,2-Ethylendiamin, 1,2- und 1,3-Diaminopropan, 1,4-Diaminobutan, 1,6-Diaminohexan, Isophorondiamin (IPDA), Isomerengemisch von 2,2,4- und 2,4,4-Trimethylhexamethylendiamin, 2-Methylpentamethylendiamin, Diethylentriamin, 4,4-Diaminodicyclohexylmethan und/oder Dimethylethylendiamin oder Mischungen aus mindestens zwei hiervon eingesetzt werden.

Bevorzugt ist die Komponente C1) ausgewählt aus der Gruppe bestehend aus 1,2-Ethylendiamin, Bis(4-aminocyclohexyl)methan, 1,4-Diaminobutan, IPDA, Ethanolamin, Diethanolamin und Diethylentriamin oder einer Mischung aus mindestens zwei hiervon.

In einer weiteren bevorzugten Ausführungsform enthält die Komponente C1) > 75 mol%, besonders bevorzugt ≥ 80 mol%, ganz besonders bevorzugt ≥ 85 mol%, weiterhin bevorzugt ≥ 95 mol% und des Weiteren bevorzugt 100 mol% 1,2-Ethylendiamin oder IPDA oder ein Gemisch aus 1,2-Ethylendiamin und IPDA, wobei die Summe der beiden Amine in Bezug auf die Gesamtmenge an C1) bevorzugt in den genannten Bereichen vorliegt. Bevorzugt enthält die Komponente C1) > 75 mol%, besonders bevorzugt ≥ 80 mol%, ganz besonders bevorzugt ≥ 85 mol%, weiterhin bevorzugt ≥ 95 mol% und des Weiteren bevorzugt 100 mol% 1,2-Ethylendiamin.

Bevorzugt umfasst die hydrophilierende Komponente C2) mindestens eine anionisch hydrophilierende Verbindung. Weiterhin bevorzugt beinhaltet die hydrophilierende Komponente C2) eine anionisch hydrophilierende Verbindung zu mindestens 80 Gew.-%, oder bevorzugt zu mindestens 90 Gew.-%, bezogen auf das Gesamtgewicht der Komponente C2). Besonders bevorzugt besteht die Komponente C2) aus ausschließlich anionisch hydrophilierenden Verbindungen.

Geeignete anionisch hydrophilierende Verbindungen enthalten wenigstens eine anionische oder ionogene Gruppe, die in eine anionische Gruppe überführt werden kann. Des Weiteren bevorzugt weisen geeignete anionisch hydrophilierende Verbindungen wenigstens zwei Aminogruppen und besonders bevorzugt zwei Aminogruppen auf. Besonders bevorzugt umfasst die hydrophilierende Komponente C2) eine anionisch hydrophilierende Verbindung, die wenigstens eine anionische oder ionogene Gruppe und wenigstens zwei Aminogruppen aufweist oder besteht daraus.

Geeignete anionisch hydrophilierende Verbindungen als Komponente C2), im Weiteren auch Hydrophilierungsmittel C2) genannt, enthalten bevorzugt eine Sulfonsäure- oder Sulfonatgruppe, besonders bevorzugt eine Natriumsulfonatgruppe. Geeignete anionisch hydrophilierende Verbindungen als Komponente C2) sind insbesondere die Alkalimetallsalze der Mono- und Diaminosulfonsäuren. Beispiele solcher anionischen Hydrophilierungsmittel sind Salze der 2-(2-Aminoethylamino)ethansulfonsäure, Ethylendiamin-propyl- oder -butylsulfonsäure oder 1,2- oder 1,3-Propylendiamin-β-ethylsulfonsäure oder Mischungen aus mindestens zwei hiervon.

Besonders bevorzugte anionische Hydrophilierungsmittel C2) sind solche, die Sulfonatgruppen als ionische Gruppen und zwei Aminogruppen enthalten, wie die Salze der 2-(2-Aminoethylamino)ethylsulfonsäure und 1,3-Propylendiamin-β-ethylsulfonsäure. Ganz besonders bevorzugt wird 2-(2-Aminoethylamino)ethylsulfonsäure oder deren Salze als anionisches Hydrophilierungsmittel C2) eingesetzt.

Gegebenenfalls kann die anionische Gruppe in der Komponente C2) auch eine Carboxylat- bzw. Carbonsäuregruppe sein. Die Komponente C2) wird dann bevorzugt aus Diaminocarbonsäuren ausgewählt. Bei dieser alternativen Ausführungsform müssen jedoch die Carbonsäure-basierenden Komponenten C2) in höheren Konzentrationen eingesetzt werden, verglichen mit solchen Komponenten C2), die Sulfonat- oder Sulfonsäuregruppen tragen. Besonders bevorzugt werden daher zur Herstellung des Polyurethanharnstoffs keine hydrophilierenden Verbindungen, die ausschließlich Carboxylatgruppen als anionische Gruppen der Komponente C2) tragen eingesetzt.

In einer bevorzugten Ausführungsform wird zur Herstellung des erfindungsgemäß verwendeten Polyurethanharnstoffs in einem Bereich von ≥ 0,1 und ≤ 10 Gew.-% der Komponente C2) und besonders bevorzugt in einem Bereich von ≥ 0,5 und ≤ 4 Gew.-% der Komponente C2), jeweils bezogen auf die Gesamtmasse des Polyurethanharnstoffs, eingesetzt.

Zur Hydrophilierung können auch Mischungen aus anionischen Hydrophilierungsmitteln C2) und weiteren Hydrophilierungsmitteln D), welche von C2) verschieden sind, verwendet werden.

Geeignete weitere Hydrophilierungsmittel D) sind beispielsweise nichtionische hydrophilierende Verbindungen D1) und/oder hydroxyfunktionelle ionische oder ionogene Hydrophilierungsmittel D2). Bevorzugt handelt es sich bei der Komponente D) um nichtionisch hydrophilierende Komponenten D1).

Geeignete hydroxyfunktionelle ionische oder ionogene Hydrophilierungsmittel als Komponente D2) sind beispielsweise Hydroxycarbonsäuren wie Mono- und Dihydroxycarbonsäuren, wie 2-Hydroxyessigsäure, 3-Hydroxypropansäure, 12-Hydroxy-9-octadecansäure (Rizinolsäure), Hydroxypivalinsäure, Milchsäure, Dimethylolbuttersäure und/oder Dimethylolpropionsäure oder Gemische von mindestens zwei hieraus. Bevorzugt sind Hydroxypivalinsäure, Milchsäure und/oder Dimethylolpropionsäure, besonders bevorzugt ist Dimethylolpropionsäure. Bevorzugt werden keine hydroxyfunktionellen ionische oder ionogenen Hydrophilierungsmittel D2), insbesondere bevorzugt keine Hydrophilierungsmittel, die Carboxylat und Hydroxygruppen aufweisen, wie beispielsweise Dimethylolpropionsäure eingesetzt. Bevorzugt ist die Menge an hydroxyfunktionellen ionischen oder ionogenen Hydrophilierungsmittel D2) in einem Bereich von 0 bis 1 Gew.-%, oder bevorzugt in einem Bereich von 0 bis 0,5 Gew.-%, bezogen auf die Gesamtmasse des Polyurethanharnstoffs in dem Polyurethanharnstoff enthalten.

Geeignete nichtionisch hydrophilierende Verbindungen als Komponente D1) sind z.B. Polyoxyalkylenether, welche über isocyanatreaktive Gruppen, wie Hydroxy-, Amino- oder Thiolgruppen verfügen. Bevorzugt sind monohydroxyfunktionelle, im statistischen Mittel 5 bis 70, bevorzugt 7 bis 55 Ethylenoxideinheiten pro Molekül aufweisenden Polyalkylenoxidpolyetheralkohole, wie sie in an sich bekannter Weise durch Alkoxylierung geeigneter Startermoleküle zugänglich sind (z.B. in Ullmanns Encyclopädie der technischen Chemie, 4. Auflage, Band 19, Verlag Chemie, Weinheim S. 31-38). Diese sind entweder reine Polyethylenoxidether oder gemischte Polyalkylenoxidether, wobei sie mindestens 30 mol-%, bevorzugt mindestens 40 mol-% bezogen auf alle enthaltenen Alkylenoxideinheiten an Ethylenoxideinheiten enthalten.

Besonders bevorzugte nichtionische Verbindungen sind monofunktionelle gemischte Polyalkylenoxidpolyether, die 40 bis 100 mol-% Ethylenoxid- und 0 bis 60 mol-% Propylenoxideinheiten aufweisen.

Geeignete Startermoleküle für solche nichtionischen Hydrophilierungsmittel sind insbesondere gesättigte Monoalkohole wie Methanol, Ethanol, n-Propanol, Isopropanol, n-Butanol, Isobutanol, sec-Butanol, die isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykol-monoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole der vorstehend genannten Art. Besonders bevorzugt werden Diethylenglykolmonobutylether, Methanol oder n-Butanol als Startermoleküle verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

In einer bevorzugten Ausführungsform der Erfindung enthält der erfindungsgemäß verwendete Polyurethanharnstoff in einem Bereich von ≥ 0 und ≤ 20 Gew.-% der Komponente D), bevorzugt in einem Bereich von ≥ 0 und ≤ 10 Gew.-% der Komponente D) und ganz besonders bevorzugt in einem Bereich von ≥ 0 und ≤ 5 Gew.-% der Komponente D), jeweils bezogen auf die Gesamtmasse des Polyurethanharnstoffs. In einer weiteren bevorzugten Ausführungsform wird Komponente D) zur Herstellung des Polyurethanharnstoffs nicht verwendet.

Als Komponente E) können wahlweise Polyole, insbesondere nicht-polymere Polyole, des genannten Molekulargewichtsbereichs von 62 bis 399 mol/g mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A (2,2-Bis(4-hydroxycyclohexyl)propan), Trimethylolpropan, Trimethylolethan, Glycerin, Pentaerythrit sowie deren beliebige Mischungen untereinander eingesetzt werden.

In einer bevorzugten Ausführungsform der Erfindung enthält der erfindungsgemäß verwendete Polyurethanharnstoff ≤ 10 Gew.-% der Komponente E), bevorzugt ≤ 5 Gew.-%, besonders bevorzugt 0 Gew.-% der Komponente E), jeweils bezogen auf die Gesamtmasse des Polyurethanharnstoffs. Bevorzugt beinhaltet der Polyurethanharnstoff die Komponente E) in einem Bereich von 0,1 bis 10 Gew.-%, bevorzugt in einem Bereich von 0,2 bis 8 Gew.-%, bevorzugt in einem Bereich von 0,1 bis 5 Gew.-%, bezogen jeweils auf die Gesamtmasse des Polyurethanharnstoffs. In einer weiteren bevorzugten Ausführungsform wird Komponente E) zur Herstellung des Polyurethanharnstoffs nicht verwendet.

In einer bevorzugten Ausführungsform wird zur Herstellung des erfindungsgemäß verwendeten Polyurethanharnstoffs in einem Bereich von ≥ 0,5 und ≤ 20 Gew.-% der Summe der Komponenten C1) und gegebenenfalls E) und besonders bevorzugt in einem Bereich von ≥ 1 und ≤ 15 Gew.-% der Summe der Komponenten C1) und gegebenenfalls E), jeweils bezogen auf die Gesamtmasse des Polyurethanharnstoffs, eingesetzt.

Als Komponente F) können weitere polymere Polyole, welche unterschiedlich sind von B) eingesetzt werden.

Beispiele sind polymere Polyole, die nicht unter die Definition von B) fallen, weil sie keine Polyetherpolyole sind - beispielsweise die in der Polyurethanlacktechnologie an sich bekannten Polyesterpolyole, Polyacrylatpolyole, Polyurethanpolyole, Polycarbonatpolyole, , Polyesterpolyacrylatpolyole, Polyurethanpolyacrylatpolyole, Polyurethanpolyesterpolyole, , Polyurethanpolycarbonatpolyole und Polyesterpolycarbonatpolyole.

Bevorzugt handelt es sich bei der Komponente F) nicht um polymere Polyole die Estergruppen aufweisen, insbesondere nicht um Polyesterpolyole.

Die Komponenten B) und F) enthalten erfindungsgemäß zusammen ≤ 30 Gew.-%, bevorzugt ≤ 10 Gew.-%, und besonders bevorzugt ≤ 5 Gew.-%, an Komponente F), bezogen auf die Gesamtmasse der Komponenten B) und F). Ganz besonders bevorzugt wird die Komponente F) zur Herstellung des Polyurethanharnstoffs nicht eingesetzt.

In einer bevorzugten Ausführungsform wird zur Herstellung des erfindungsgemäß verwendeten Polyurethanharnstoffs in einem Bereich von ≥ 55 und ≤ 90 Gew.-% der Summe der Komponenten B) und gegebenenfalls F) und besonders bevorzugt in einem Bereich von ≥ 60 und ≤ 85 Gew.-% der Summe der Komponenten B) und gegebenenfalls F), jeweils bezogen auf die Gesamtmasse des Polyurethanharnstoffs, eingesetzt.

Bei der Komponente G) handelt es sich um Verbindungen, die genau eine isocyanatreaktive Gruppe aufweisen, oder um Verbindungen, die mehr als eine isocyanatreaktive Gruppe aufweisen, wobei nur eine der isocyanat reaktiven Gruppen unter den gewählten Umsetzungsbedingungen mit den in der Reaktionsmischung vorhandenen Isocyanatgruppen reagiert.

Bei den isocyanatreaktiven Gruppen der Komponente G) kann es sich dabei um jede funktionelle Gruppe handeln, die mit einer Isocyanatgruppe reagieren kann, wie beispielsweise Hydroxygruppen, Thiolgruppen oder primäre und sekundäre Aminogruppen.

Isocyanatreaktive Gruppen im Sinne der Erfindung sind dabei primäre oder sekundäre Aminogruppen, die mit Isocyanatgruppen unter Bildung von Harnstoffgruppen reagieren. Neben der Aminogruppe können die Verbindungen der Komponente G) auch andere prinzipiell isocyanatreaktive Gruppen, wie OH-Gruppen aufweisen, wobei nur eine der isocyanatreaktiven Gruppen unter den gewählten Umsetzungsbedingungen mit den in der Reaktionsmischung vorhandenen Isocyanatgruppen reagiert. Dies kann beispielsweise durch Reaktion entsprechender Aminoalkohole bei relativ niedrigen Temperaturen erfolgen, beispielsweise bei 0 bis 60°C, bevorzugt bei 20 bis 40°C. Bevorzugt wird dabei in Abwesenheit von Katalysatoren gearbeitet, welche die Reaktion von Isocyanatgruppen mit Alkoholgruppen katalysieren würden.

Beispiele für geeignete Verbindungen der Komponente G) sind primäre/sekundäre Amine, wie Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, Diethanolamin, 3-Amino-1-methylaminopropan, 3-Amino-1-ethylaminopropan, 3-Amino-1-cyclohexylaminopropan, 3-Amino-1- methylaminobutan, Ethanolamin, 3-Aminopropanol oder Neopentanolamin.

Geeignete monofunktionellen Verbindungen sind auch Ethanol, n-Butanol, Ethylenglykolmonobutylether, Diethylenglykolmonomethylether, Diethylenglykolmonobutylether, Propylenglykolmonomethylether, Dipropylenglykol-monomethylether, Tripropylenglykolmonomethylether, Dipropylenglykolmono-propylether, Propylenglykolmonobutylether, Dipropylenglykolmonobutylether, Tripropylenglykolmonobutylether, 2-Ethylhexanol, 1-Octanol, 1-Dodecanol, 1-Hexadecanol.

In einer bevorzugten Ausführungsform wird zur Herstellung des erfindungsgemäß verwendeten Polyurethanharnstoffs ≥ 0,1 und ≤ 20 Gew.-% der Komponente G) und besonders bevorzugt ≥ 0,3 und ≤ 10 Gew.-% der Komponente G), jeweils bezogen auf die Gesamtmasse des Polyurethanharnstoffs, eingesetzt.

Erfindungsgemäß wird die Komponente H) eingesetzt und das molare Verhältnis von Komponente G) zu Komponente H) beträgt bevorzugt 5:1 bis 1:5, besonders bevorzugt 1,5:1 bis 1:4 und ganz besonders bevorzugt 1:1 bis 1:3.

In einer bevorzugten Ausführungsform werden zur Herstellung der erfindungsgemäß verwendeten Polyurethanharnstoffe die Komponenten A) bis H) in den folgenden Mengen eingesetzt, wobei sich die Einzelmengen stets zu 100 Gew.-% addieren:
5 bis 40 Gew.-% Komponente A),
55 bis 90 Gew.-% Summe der Komponenten B) und gegebenenfalls F),
0,5 bis 20 Gew.-% Summe der Komponenten C1) und gegebenenfalls E),
0,1 bis 10 Gew.-% Komponente C2),
0 bis 20 Gew.-% Komponente D),
0,1 bis 20 Gew.-% der Komponente G) und
0 bis 10 Gew.-% Komponente H).

In einer besonders bevorzugten Ausführungsform werden zur Herstellung der erfindungsgemäß verwendeten Polyurethanharnstoffe die Komponenten A) bis H) in den folgenden Mengen eingesetzt, wobei sich die Einzelmengen stets zu 100 Gew.-% aufaddieren:
10 bis 35 Gew.-% Komponente A),
60 bis 85 Gew.-% Summe der Komponenten B) und gegebenenfalls F),
1 bis 15 Gew.-% Summe der Komponenten C1) und gegebenenfalls E),
0,5 bis 4 Gew.-% Komponente C2),
0 bis 10 Gew.-% Komponente D),
0,3 bis 10 Gew.-% der Komponente G) und
0,1 bis 3 Gew.-% Komponente H).

In einer besonders bevorzugten Ausführungsform der Erfindung umfasst das kontaktklebende Produkt einen Polyurethanharnstoff, der erhältlich ist, durch Umsetzung von wenigstens
A) einer aliphatischen Polyisocyanat-Komponente, wobei es sich um ein Gemisch aus IPDI und HDI handelt,
B) einer polymeren Polyether-Polyol-Komponente, welche ein Gemisch aus wenigstens zwei Poly(tetramethylenglykol)polyetherpolyolen ist und wobei sich die Poly(tetramethylenglykol)polyetherpolyole in ihren zahlenmittleren Molekulargewichten unterscheiden,
C) einer aminofunktionellen Kettenverlängerer-Komponente mit 2 isocyanatreaktiven primären und/oder sekundären Aminogruppen, enthaltend wenigstens eine aminofunktionelle Verbindung C1), die keine ionischen oder ionogenen Gruppen aufweist und/oder eine aminofunktionelle Verbindung C2), die ionische oder ionogene Gruppen aufweist,
D) gegebenenfalls weiteren hydrophilierenden Komponenten, die unterschiedlich sind von C2), wobei es sich um nichtionisch hydrophilierende Komponenten D1) handelt,
E) gegebenenfalls hydroxyfunktionellen Verbindungen mit einem Molekulargewicht von 62 bis 399 mol/g,
F) gegebenenfalls weitere polymere Polyole, welche unterschiedlich sind von B),
G) einer Verbindung, die genau eine isocyanatreaktive Gruppe aufweist, oder einer Verbindung, die mehr als eine isocyanatreaktive Gruppe aufweist, wobei nur eine der isocyanat reaktiven Gruppen unter den gewählten Umsetzungsbedingungen mit den in der Reaktionsmischung vorhandenen Isocyanatgruppen reagiert, wobei es sich bei der isocyanat reaktiven Gruppe um eine primäre und/oder sekundäre Amino- und/oder Hydroxygruppe handelt und
H) einer aliphatischen Polyisocyanat-Komponente mit einer mittleren Isocyanatfunktionalität von > 2,6 und ≤ 4, wobei die Komponente H) aus einem aliphatischen oder cycloaliphatischen Polyisocyanat-Oligomer mit Isocyanurat-, Urethan-, Allophanat-, Biuret-, Iminooxadiazindion- oder Oxadiazintrionstruktur, basierend auf HDI, IPDI und/oder H12-MDI besteht,
wobei die Komponenten B) und F) zusammen ≤ 30 Gew.-% an Komponente F), bezogen auf die Gesamtmasse der Komponenten B) und F) enthalten.

Ganz besonders bevorzugt ist der erfindungsgemäß verwendete Polyurethanharnstoff erhältlich durch Umsetzung ausschließlich der Komponenten A) bis H). Es werden dann keine weiteren Komponenten zur Herstellung des Polyurethanharnstoffs eingesetzt.

Das zahlenmittlere Molekulargewicht der erfindungsgemäß bevorzugt verwendeten Polyurethanharnstoffe beträgt bevorzugt von ≥ 2000 bis ≤ 300000 g/mol, bevorzugt von ≥ 5000 bis ≤ 150000 g/mol.

Der erfindungsgemäß verwendete Polyurethanharnstoff ist bevorzugt amorph und weist einen Tg ≤ - 25 °C, besonders bevorzugt von ≤ - 50 °C und ganz besonders bevorzugt von ≤ - 70 °C auf.

Amorph bedeutet im Sinne dieser Erfindung, dass der Polyurethanharnstoff im in der im folgenden ausgeführten Messmethode genannten Temperaturbereich keine oder nur so geringe kristalline Anteile ausbildet, dass mittels der beschriebenen DSC Messungen nur ein oder mehrere Glasübergangspunkte T_{g}, aber keine Schmelzbereiche mit einer Schmelzenthalpie ≥ 20J/g in dem genannten Temperaturbereich gefunden werden können.

Die Glasübergangstemperatur T_{g} wird im Rahmen dieser Erfindung mittels dynamischer Differenzkalorimetrie in Anlehnung an die DIN EN 61006, Verfahren A, bestimmt, wobei ein DSC Gerät verwendet wird, das zur Bestimmung von T_{g} mit Indium und Blei kalibriert ist und wobei drei unmittelbar aufeinander folgende Durchläufe einer Aufheizungen von - 100°C bis +150°C, bei einer Heizrate von 20 K/min, mit anschließender Abkühlung mit einer Kühlrate von 320 K/min vorgenommen und die dritte Aufheizkurve zur Bestimmung der Werte verwendet wird und wobei als T_{g} die Temperatur bei der halben Höhe einer Glasübergangsstufe bestimmt wird.

Sollte der Polyurethanharnstoff in Form einer Dispersion vorliegen, wird bei der Probenpräparation für die DSC Messungen besonders vorgegangen. Bei der Bestimmung der Glasübergangstemperatur T_{g} von Dispersionen mittels DSC kann die T_{g} des Polymers durch die kalorischen Effekte des Dispergens (Wasser, Neutralisationsmittel, Emulgatoren, Colöser, etc.) maskiert bzw. wegen der Mischbarkeit mit dem Polymer deutlich abgesenkt werden. Daher wird das Dispergens vor der DSC-Messung bevorzugt durch geeignete Trocknung zunächst vollständig entfern, denn auch geringe Restmengen Dispergens wirken als Weichmacher und können die Glastemperatur dadurch absenken. Die Dispersion wird daher bevorzugt mit 100 µm Nassschichtdicke (NSD) auf eine Glasplatte gerakelt, abgelüftet und dann für zwei Tage in einer Trockenbox bei RT und 0% relativer Luftfeuchtigkeit (rF) schonend getrocknet. Nach dieser Probenpräparation kann in der ersten Aufheizung der DSC Messung noch einen breiten endothermen Verdampfungsbereich von Restfeuchte im Film. Um die bestimmten Werte möglichst von solchen Einflüssen frei zu erhalten, wir daher die dritte Aufheizkurve ausgewertet.

Der erfindungsgemäß zur Herstellung des Produktes eingesetzte Polyurethanharnstoff liegt bevorzugt in einem physiologisch akzeptablen Medium vor. Besonders bevorzugt handelt es sich bei dem Medium um Wasser und ganz besonders bevorzugt liegt der Polyurethanharnstoff als wässrige Dispersion vor. Wasser bildet im Allgemeinen, neben wahlweise vorhandenen anderen flüssigen Medien, wie beispielsweise Lösungsmitteln, den Hauptbestandteil (> 50 Gew.-%) des Dispergiermediums, bezogen auf die Gesamtmenge des flüssigen Dispergiermediums, gegebenenfalls auch das alleinige flüssige Dispergiermedium.

Das erfindungsgemäße Produkt selbst enthält den Polyurethanharnstoff an sich, der keine oder nur noch Restmengen dieses Mediums enthält.

Bevorzugt ist der verwendete Polyurethanharnstoff daher in Wasser dispergierbar, was im Rahmen dieser Erfindung bedeutet, dass der Polyurethanharnstoff bei 23°C eine sedimentationsstabile Dispersion in Wasser, insbesondere entionisiertem Wasser, bilden kann.

Die erfindungsgemäß verwendeten Polyurethanharnstoffe sind bevorzugt erhältlich, indem isocyanatfunktionelle Polyurethanpräpolymere a) aus den Komponenten A), B) und gegebenenfalls D) und/oder C2), sowie gegebenenfalls den Verbindungen E) und/oder H) hergestellt werden (Schritt a) und deren freie NCO-Gruppen anschließend ganz oder teilweise mit der aminofunktionellen Kettenverlängerer-Komponente C), sowie der Komponente G) und gegebenenfalls der Komponente D) und H) umgesetzt werden (Schritt b)).

Wobei wenn die Komponente H) erst in Schritt b) eingesetzt wird, diese bevorzugt vor der Zugabe der Komponente C) zugegeben und mit dem Prepolymer a) umgesetzt wird.

In einer bevorzugten Ausführungsform der Erfindung erfolgt im Schritt b) die Umsetzung mit einem Diamin oder mehreren Diaminen (Komponente C) unter Kettenverlängerung wobei außerdem die monofunktionelle Komponente G) als Kettenabbrecher zur Molekulargewichtssteuerung zugesetzt wird.

Die Komponenten A) bis H) sind dabei wie oben angegeben definiert. Es gelten auch die oben genannten bevorzugten Ausführungsformen.

Bevorzugt wird im Schritt b) der Umsetzung des Präpolymers a) zur Herstellung des Polyurethanharnstoffs eine Mischung aus Komponenten C1), C2) und G) zur Umsetzung gebracht. Durch die Verwendung der Komponente C1) kann eine hohe Molmasse aufgebaut werden, ohne dass die Viskosität des zuvor hergestellten isocyanatfunktionellen Präpolymers in einem Maße ansteigt, welches einer Verarbeitung entgegenstehen würde. Durch die Verwendung der Kombination der Komponenten C1), C2) und G) lässt sich eine optimale Balance zwischen Hydrophilie und Kettenlänge einstellen.

Bevorzugt weist das erfindungsgemäß verwendete Polyurethanpräpolymere a) endständige Isocyanatgruppen auf, d.h. die Isocyanatgruppen liegen an den Kettenenden des Präpolymers. Besonders bevorzugt weisen sämtliche Kettenenden des Präpolymers Isocyanatgruppen auf.

Über die hydrophilierende Komponente C2) und/oder D) kann die Hydrophilie des Präpolymers gesteuert werden. Daneben spielen für die Hydrophilie des Präpolymers natürlich auch noch weitere Komponenten, speziell auch die Hydrophilie der Komponente B) eine Rolle.

Bevorzugt sind die isocyanatfunktionellen Polyurethanpräpolymere a) wasserunlöslich und nicht in Wasser dispergierbar.

Im Rahmen der Erfindung bedeutet der Begriff "wasserunlösliches, nicht wasserdispergierbares Polyurethanpräpolymer" insbesondere, dass die Wasserlöslichkeit des erfindungsgemäß verwendeten Präpolymers bei 23°C weniger als 10 g / Liter, bevorzugter weniger als 5 g / Liter beträgt und das Präpolymer bei 23° keine sedimentationsstabile Dispersion in Wasser, insbesondere entionisiertem Wasser, ergibt. Mit anderen Worten setzt sich das Präpolymer, beim Versuch es in Wasser zu dispergieren, ab. Die Wasserunlöslichkeit bzw. fehlende Dispergierbarkeit in Wasser bezieht sich auf entionisiertes Wasser ohne Zusatz von Tensiden.

Weiterhin weist das erfindungsgemäß verwendete Polyurethanpräpolymere a) bevorzugt im Wesentlichen weder ionische noch ionogene (zur Bildung von ionischen Gruppen befähigte) Gruppen auf. Im Rahmen der vorliegenden Erfindung bedeutet dies, dass der Anteil der ionischen und/oder ionogenen Gruppen, wie insbesondere anionischer Gruppen, wie Carboxylat oder Sulfonat, oder kationischer Gruppen weniger beträgt als 15 Milliequivalente pro 100 g Polyurethanpräpolymer a1), bevorzugt weniger als 5 Milliequivalente, besonders bevorzugt weniger als 1 Milliequivalent und ganz besonders bevorzugt weniger als 0,1 Milliequivalente pro 100 g Polyurethanpräpolymer a).

Im Falle saurer ionischer und/oder ionogener Gruppen beträgt die Säurezahl des Präpolymers zweckmäßig unter 30 mg KOH/g Präpolymer, bevorzugt unter 10 mg KOH/g Präpolymer. Die Säurezahl gibt die Masse Kaliumhydroxid in mg an, die zur Neutralisation von 1 g der zu untersuchenden Probe erforderlich ist (Messung nach DIN EN ISO 211). Die neutralisierten Säuren, also die entsprechenden Salze weisen naturgemäß keine oder eine reduzierte Säurezahl auf. Hier ist erfindungsgemäß die Säurezahl der korrespondierenden freien Säure entscheidend.

Dabei sind die wasserunlöslichen, nicht wasserdispergierbaren, isocyanatfunktionellen Polyurethanpräpolymeren a) bevorzugt ausschließlich erhältlich aus den Komponenten A), B) und gegebenenfalls D), E) und/oder H).

Die Komponenten sind dabei wie oben angegeben definiert. Es gelten auch die oben genannten bevorzugten Ausführungsformen.

Es werden folglich bevorzugt in dieser Ausführungsform keine ionisch hydrophilierenden Komponenten C2) oder auch D2) zur Herstellung des Präpolymers a) eingesetzt. Auch wird die Komponente G) in diesem Schritt nicht zugesetzt. Die Hydrophilierungsmittel D1) werden bevorzugt in solchen Mengen eingesetzt, dass das Präpolymer dennoch wasserunlöslich und nicht wasserdispergiebar ist. Besonders bevorzugt werden ≤ 10 Gew.-% der Komponente D1), ganz besonders bevorzugt ≤ 5 Gew.-% und weiterhin bevorzugt ≤ 2 Gew.-% der Komponente D1), jeweils bezogen auf die Gesamtmasse des Polyurethanharnstoffs, eingesetzt. Weiterhin bevorzugt wird die Komponente D1) zur Herstellung des Präpolymers a) nicht eingesetzt.

Für diese Ausführungsform der Erfindung weist die Komponente B) weder ionische noch ionogene Gruppen auf. Weiterhin werden bei dieser Ausführungsform der Erfindung als Komponente B) bevorzugt nur Polyetherpolyole, insbesondere Polyalkylenoxidether eingesetzt, die ≤ 10 mol-% und bezogen auf alle enthaltenen Alkylenoxideinheiten an Ethylenoxideinheiten und bevorzugt keine Ethylenoxideinheiten enthalten.

Die in dieser Ausführungsform der Erfindung bevorzugt verwendeten Polyurethanharnstoffe weisen folglich ionische oder ionogene Gruppen auf, bevorzugt anionische Gruppen auf, diese anionischen Gruppen werden dabei in die erfindungsgemäß verwendeten Polyurethanharnstoffe über die in Schritt b) eingesetzte hydrophilierende Komponente C2) eingeführt. Die erfindungsgemäß verwendeten Polyurethanharnstoffe weisen gegebenenfalls zusätzlich nicht-ionische Komponenten zu Hydrophilierung auf.

Besonders bevorzugt sind in den erfindungsgemäß verwendeten Polyurethanharnstoffen zur Hydrophilierung ausschließlich Sulfonatgruppen enthalten, welche in Schritt b) über entsprechende Diamine als Komponente C2) in den Polyurethanharnstoff eingeführt werden.

In einer alternativen, weniger bevorzugten Ausführungsform der Erfindung sind die zur Herstellung des erfindungsgemäßen Polyurethanharnstoffs eingesetzten Präpolymere a) wasserlöslich oder wasserdispergierbar. In dieser Ausführungsform werden die hydrophilierende Komponente D) und/oder C2) bei der Herstellung des Präpolymers a) in einer Menge eingesetzt, die ausreichend ist, damit das Präpolymer wasserlöslich oder wasserdispergierbar ist. Das Präpolymer a) weist dabei bevorzugt ionische oder ionogene Gruppen auf.

Geeignete hydrophilierende Komponenten D) und C2) sind für diese Ausführungsform der Erfindung die oben für D) und C2) genannten Verbindungen. Bevorzugt werden als hydrophilierende Komponenten wenigstens die oben unter D1) und/oder C2) genannten Verbindungen eingesetzt.

Die zur Herstellung der erfindungsgemäßen Produkte verwendeten Polyurethanharnstoffe werden bevorzugt vor, während oder nach Schritt b), besonders bevorzugt während oder nach Schritt b) in Wasser dispergiert. So wird eine Dispersion der Polyurethanharnstoffe erhalten.

Die Herstellung der Polyurethanharnstoff-Dispersionen kann dabei in einer oder mehreren Stufe/-n in homogener oder bei mehrstufiger Umsetzung, teilweise in disperser Phase durchgeführt werden. Nach Herstellung des Präpolymers a) erfolgt bevorzugt ein Dispergier-, Emulgier- oder Lösungsschritt. Im Anschluss erfolgt gegebenenfalls eine weitere Polyaddition oder Modifikation in disperser Phase. Dabei wird jeweils das für das entsprechende Präpolymer geeignete Löse- oder Dispergiermittel wie beispielsweise Wasser oder Aceton oder Mischungen daraus gewählt.

Dabei können alle aus dem Stand der Technik bekannten Verfahren wie z. B. Präpolymer-Mischverfahren, Acetonverfahren oder Schmelzdispergierverfahren verwendet werden. Bevorzugt wird das Aceton-Verfahren angewandt.

Für die Herstellung nach dem Aceton-Verfahren werden üblicherweise die Bestandteile B), gegebenenfalls D) und E) und die Polyisocyanatkomp,onente A) gegebenfalls in Kombination mit der Komponente H) zur Herstellung eines isocyanatfunktionellen Polyurethanpräpolymers ganz oder teilweise vorgelegt und gegebenenfalls mit einem mit Wasser mischbaren aber gegenüber Isocyanatgruppen inerten Lösungsmittel verdünnt und auf Temperaturen im Bereich von 50 bis 120°C aufgeheizt. Zur Beschleunigung der Isocyanatadditionsreaktion können die in der Polyurethan-Chemie bekannten Katalysatoren eingesetzt werden.

Geeignete Lösungsmittel sind die üblichen aliphatischen, ketofunktionellen Lösemittel wie Aceton, 2-Butanon, die nicht nur zu Beginn der Herstellung, sondern gegebenenfalls in Teilen auch später zugegeben werden können. Bevorzugt sind Aceton und 2-Butanon, besonders bevorzugt ist Aceton. Auch die Zugabe anderer Lösemittel ohne isocyanatreaktive Gruppen ist möglich, jedoch nicht bevorzugt.

Anschließend können die gegebenenfalls zu Beginn der Reaktion noch nicht zugegebenen Bestandteile von A), B) und gegebenenfalls H), D) und E) zu dosiert werden.

Bei der Herstellung des Polyurethan-Präpolymeren aus A), B) und gegebenenfalls H), D) und E) beträgt das Stoffmengenverhältnis von Isocyanat-Gruppen zu isocyanatreaktiven Gruppen bevorzugt 1,05 bis 3,5, besonders bevorzugt 1,1 bis 3,0 und ganz besonders bevorzugt 1,1 bis 2,5.

Die Umsetzung der Komponenten A), B) und gegebenenfalls H), D) und E) zum Präpolymer kann teilweise oder vollständig, bevorzugt aber vollständig erfolgen. Es können so Polyurethanpräpolymere, die freie Isocyanatgruppen enthalten, in Substanz oder in Lösung erhalten werden.

Sollten ionogene Gruppen, wie beispielsweise Carboxylgruppen, im Präpolymer vorliegen können diese in einem weiteren Schritt durch Neutralisation in ionische Gruppen überführt werden.

Im Neutralisationsschritt zur teilweisen oder vollständigen Überführung potentiell anionischer Gruppen in anionische Gruppen können Basen wie tertiäre Amine, z.B. Trialkylamine mit 1 bis 12, bevorzugt 1 bis 6 C-Atomen, besonders bevorzugt 2 bis 3 C-Atomen in jedem Alkylrest oder ganz besonders bevorzugt Alkalimetallbasen wie die entsprechenden Hydroxide eingesetzt werden.

Als Neutralisationsmittel sind bevorzugt anorganische Basen, wie wässrige Ammoniaklösung oder Natrium- bzw. Kaliumhydroxid einsetzbar, besonders bevorzugt sind Natriumhydroxid und Kaliumhydroxid.

Die Stoffmenge der Basen beträgt bevorzugt 50 und 125 mol%, besonders bevorzugt zwischen 70 und 100 mol% der Stoffmenge der zu neutralisierenden Säuregruppen. Die Neutralisation kann, auch gleichzeitig mit der Dispergierung erfolgen, in dem das Dispergierwasser bereits das Neutralisationsmittel enthält.

Im Anschluss an die Neutralisation wird in einem weiteren Verfahrensschritt, falls noch nicht oder nur teilweise geschehen, das erhaltene Präpolymer mit Hilfe von aliphatischen Ketonen wie Aceton oder 2-Butanon gelöst.

Bei der Kettenverlängerung/-terminierung in Stufe b) werden die Komponenten C), G) und gegebenenfalls D) mit den noch verbliebenen Isocyanatgruppen des Präpolymers umgesetzt. Bevorzugt wird die Kettenverlängerung/-terminierung vor der Dispergierung in Wasser durchgeführt.

Geeignete Komponenten C) zur Kettenverlängerung sowie G) zum Kettenabbruch sind bereits oben aufgeführt. Es gelten analog auch die oben genannten bevorzugten Ausführungsformen.

Werden zur Kettenverlängerung anionische Hydrophilierungsmittel entsprechend der Definition C2) mit NH₂- oder NH-Gruppen eingesetzt, erfolgt die Kettenverlängerung der Präpolymere in Schritt b) bevorzugt vor der Dispergierung in Wasser.

Das Äquivalentverhältnis von NCO-reaktiven Gruppen der zur Kettenverlängerung und Kettenterminierung eingesetzten Verbindungen zu freien NCO-Gruppen des Präpolymers, liegt im allgemeinen zwischen 40 und 150 %, bevorzugt zwischen 50 und 110 %, besonders bevorzugt zwischen 60 und 100 %.

Die Komponenten C1), C2) und G), können gegebenenfalls in wasser- oder lösemittelverdünnter Form im erfindungsgemäßen Verfahren einzeln oder in Mischungen eingesetzt werden, wobei grundsätzlich jede Reihenfolge der Zugabe möglich ist.

Wenn Wasser oder organische Lösemittel als Verdünnungsmittel in Schritt b) mit verwendet werden, so beträgt der jeweilige Verdünnungsmittelgehalt in den eingesetzten Komponenten C1), C2) und G) bevorzugt 40 bis 95 Gew.-%.

Die Dispergierung erfolgt bevorzugt im Anschluss an die Kettenverlängerung und Kettenterminierung. Dazu wird das gelöste (beispielsweise in Aceton) und mit dem Aminen umgesetzte Polyurethanpolymer gegebenenfalls unter starker Scherung, wie z.B. starkem Rühren, entweder in das Dispergierwasser eingetragen oder es wird umgekehrt das Dispergierwasser zu den kettenverlängerte Polyurethanpolymerlösungen gerührt. Bevorzugt wird das Wasser in das gelöste Polyurethanpolymer gegeben.

Das in den Dispersionen nach dem Dispergierschritt noch enthaltene Lösemittel wird üblicherweise anschließend destillativ entfernt. Eine Entfernung bereits während der Dispergierung ist ebenfalls möglich.

Die erhaltenen wässrigen Polyurethanharnstoffdispersionen weisen bevorzugt einen Gehalt an flüchtigen organischen Verbindungen (VOC), wie beispielsweise flüchtige organische Lösemitteln, von weniger als 10 Gew.-%, bevorzugter von weniger als 3 Gew.-%, noch bevorzugter von weniger als 1 Gew.-% bezogen auf die auf wässrige Polyurethanharnstoffdispersion auf. VOCs im Sinne dieser Erfindung sind insbesondere organische Verbindung mit einem Anfangssiedepunkt von höchstens 250 °C bei einem Standarddruck von 101,3 kPa.

Die Bestimmung des Gehalts an flüchtigen organischen Verbindungen (VOC) erfolgt im Rahmen der vorliegenden Erfindung insbesondere durch gaschromatographische Analyse.

Der Polyurethanharnstoff wird zur Herstellung des Produkts bevorzugt als wässrige Dispersion eingesetzt.

Der pH-Wert der erfindungsgemäß verwendeten wässrigen Polyurethan-Dispersionen beträgt typischerweise weniger als 9,0, bevorzugt weniger als 8,5 und liegt besonders bevorzugt zwischen 5,5 und 8,0.

Um eine gute Sedimentationsstabilität zu erreichen, liegt die zahlenmittlere Teilchengröße der speziellen Polyurethanharnstoff-Dispersionen bevorzugt bei weniger als 750 nm, besonders bevorzugt bei weniger als 500 nm, bestimmt mittels Laserkorrelations-Spektroskopie nach Verdünnung mit entionisiertem Wasser (Gerät: Malvern Zetasizer 1000, Malvern Inst. Limited).

Der Feststoffgehalt der Polyurethanharnstoff-Dispersionen beträgt bevorzugt 10 bis 70 Gew.-%, besonders bevorzugt 20 bis 60 Gew.-%und ganz besonders bevorzugt 40 bis 60 Gew.-%. Die Feststoffgehalte werden ermittelt durch Erhitzen einer ausgewogenen Probe auf 125°C bis zur Gewichtskonstanz. Bei Gewichtskonstanz wird durch erneutes Auswiegen der Probe der Festkörpergehalt berechnet.

Bevorzugt weisen diese Polyurethanharnstoff-Dispersionen weniger als 5 Gew.-%, besonders bevorzugt weniger als 0,2 Gew.-%, bezogen auf die Masse der Dispersionen, an ungebundenen organischen Aminen auf.

Die zur Herstellung der erfindungsgemäßen Produkte eingesetzte Polyurethanharnstoff-Dispersionen weist bei 23°C bei einer konstanten Scherrate von 10 s⁻¹ bevorzugt eine Viskosität von ≥ 1 und ≤ 10000 mPa s, besonders bevorzugt von ≥ 10 und ≤ 5000 mPa s und ganz besonders bevorzugt von ≥ 100 und ≤ 4000 mPa s auf. Die Viskosität wird dabei wie im Methodenteil beschrieben bestimmt.

Weiterhin umfasst das kontaktklebende Produkt erfindungsgemäß ein Substrat.

Typischerweise werden als Substrate geeignete Textilien mit ausreichender Elastizität und geeigneten mechanischen Eigenschaften verwendet.

Als Substrate eignen sich bevorzugt textile Flächengebilde. Besonders bevorzugt sind faserartige Materialien mit einer nicht glatten Oberfläche als Substrat für die erfindungsgemäß verwendeten Polyurethanharnstoffe. Unter textilen Flächengebilden im Sinne der vorliegenden Erfindung sind beispielsweise Gewebe, Gewirke, Geflechte, Geschlinge, Gestricke, gebundene und ungebundene Vliese zu verstehen. Bevorzugt sind Geflechte, insbesondere solche aus Kett- und Schussfäden, Gewirke, insbesondere gekreppte Gewirke, oder Vliese.

Die textilen Flächengebilde können aus synthetischen, natürlichen Fasern und/oder deren Mischungen aufgebaut sein. Beispiele für natürliche Fasern sind Cellulose, Baumwolle, Leinen und deren chemisch modifizierte Fasern. Beispiele für synthetische Fasern sind Polyamid, Polyester usw. Grundsätzlich sind Textilien aus beliebigen Fasern für das erfindungsgemäße Verfahren geeignet. Auch Mischungen aus verschiedenen Fasern sind geeignet. Besonders bevorzugt ist die Verwendung eines geringen Anteils, insbesondere zwischen1 und 10 Gew.-% einer elastischen Faser, ganz besonders bevorzugt ist hier die Verwendung von Elasthan. Durch die erfindungsgemäß verwendeten Polyurethanharnstoffe können die Substrate in allen üblichen Arten behandelt bzw. veredelt werden, vorzugsweise durch Beschichten oder Verkleben der Fasern untereinander bzw. von Substraten miteinander.

Bevorzugt werden als Substrate Produkte oder Vliese aus synthetischen Fasern, Cellulose oder Baumwolle verwendet. Besonders bevorzugt werden Vliese, gekreppte Gewirke oder Geflechte auf Basis von Polyester- oder Polyamid oder deren Mischungen mit Baumwolle oder bevorzugt Cellulosefasern verwendet, die einen Anteil hochelastischer Fasern synthetischer Polymere (z.B. Elasthan bzw. Spandex) von 1 bis 10 Gew.-% aufweisen.

Bevorzugt haben diese Flächengebilde Flächengewichte von 20 bis 600 gsm (Gramm pro m²), besonders bevorzugt 25 bis 300 gsm und ganz besonders bevorzugt 28 bis 80 gsm.

Substrate werden bevorzugt in Form kontinuierlicher Binden, Bandagen oder Rollen eingesetzt. Bevorzugt werden als Substrate elastische Binden mit einem Dehnungsbereich von 30 bis 500 %, besonders bevorzugt von 60 bis 250% und ganz besonders bevorzugt von 120 bis 200%, bestimmt gemäß DIN 53835 Teil 2 (Bestimmung des zugelastischen Verhaltens von Textilien mittels mehrmaliger Zugbeanspruchung zwischen konstanten Dehngrenzen (Gesamtdehnung)), verwendet. Weiterhin weisen die Substrate bevorzugt eine Höchstzugkraft von 100 bis 500 N, besonders bevorzugt von 120 bis 350 N, bestimmt gemäß DIN EN ISO 13934-1 mittels Streifen-Zugversuch, auf.

Ganz besonders bevorzugt sind Langzugbinden mit einem Dehnungsbereich von 120 bis 200 % und einer Höchstzugkraft von 120 bis 350 N. Die verwendeten Substrate haben bevorzugt eine grobe, raue, nicht vollständig geschlossene Oberfläche. Diese lässt sich durch die Luftdurchlässigkeit im ungedehnten Zustand definieren. Luftdurchlässigkeiten von > 200 l/m² s sind bevorzugt, besonders bevorzugt ≥ 1000 l/m² s und ganz besonders bevorzugt ≥ 3000 l/m² s. Die Luftdurchlässigkeit der Substrate wird dabei bestimmt gemäß DIN EN ISO 9237.

In einer bevorzugten Ausführungsform der Erfindung bedeckt der Polyurethanharnstoff mindestens eine Fläche des Substrates an der Oberfläche, besonders bevorzugt bedeckt der Polyurethanharnstoff zwei gegenüberliegende Flächen des Substrates (Vorder- und Rückseite) an der Oberfläche. Ganz besonders bevorzugt bedeckt der Polyurethanharnstoff die Oberflächen dabei gleichmäßig.

Es ist dabei möglich, dass das Substrat über die vollständige Fläche und Dicke mit dem Polyurethanharnstoff imprägniert ist, bevorzugt verbleibt die Beschichtung jedoch an der Oberfläche des Substrates und dringt nicht vollständig in dessen Innenbereich (Bulk) oder die Fasern selbst ein.

Bei den erfindungsgemäßen kontaktklebenden Produkten handelt es sich bevorzugt um Produkte, die am menschlichen Körper angewendet werden. Besonders bevorzugt werden die kontaktklebenden Produkte in medizinischen Bereichen, wie insbesondere der Sportmedizin, Unfallchirurgie oder Orthopädie, sowohl zur Behandlung als auch zur Prävention von Knochen-, Gelenk- oder Muskelverletzungen oder zum Schutz und Abdeckung der Haut oder von Hautverletzungen eingesetzt. Weitere bevorzugte Anwendungen sind Bandagen für die Kompressionstherapie und die Fixierung von nicht-klebenden Wundauflagen (als "secondary dressings").

Erfindungsgemäß handelt es sich bei dem kontaktklebenden Produkt bevorzugt um ein Pflaster, einen Verband, ein Tape oder eine Bandage oder zumindest um einen Bestandteil dieser Endprodukte.

Unter Tape wird dabei im Rahmen dieser Erfindung insbesondere ein Pflasterklebeverband verstanden, der in medizinischen Bereichen, sowohl zur Behandlung, als auch zur Prävention von Knochen-, Gelenk- oder Muskelverletzungen eingesetzt werden.

Ein weiterer Gegenstand der Erfindung ist ein Verfahren zur Herstellung eines erfindungsgemäßen kontaktklebenden Produktes umfassend die Schritte
I) Auftragen des Polyurethanharnstoffs auf das Substrat in Form einer wässrigen Polyurethanharnstoff-Dispersion und
II) thermische Trocknung des behandelten Substrates bei Temperaturen bei ≥ 20°C und ≤ 200°C.

Zur Auftragung des Polyurethanharnstoffs in Schritt I wird dieser in Form einer Polyurethanharnstoff-Dispersion bevorzugt mit Zuschlagstoffen und insbesondere bevorzugt blasenfrei vermengt. Die dabei entstehende Zusammensetzung wird im Folgenden als Polyurethanharnstoffzusammensetzung bezeichnet. Zur Herstellung des kontaktklebenden Produktes wird bevorzugt eine Polyurethanharnstoffzusammensetzung umfassend den Polyurethanharnstoff in Form einer wässrigen Dispersion und weitere Zuschlagstoffe eingesetzt.

Selbstverständlich kann die Viskosität der Polyurethanharnstoff-Dispersion auf die benötigten Gegebenheiten durch Verdünnen oder Verdicken oder eine Kombination beider Methoden angepasst werden, um gewünschte Auftragsdicken zu erzielen. Dabei können als Zuschlagstoffe Verdicker Verwendung finden. Typische Verdicker sind lösliche Polymere auf Polyacrylat- oder Polyurethanbasis, wie sie aus dem Stand der Technik bekannt sind. Bevorzugt sind Verdicker auf Basis von Polyurethanpolymeren. Zur Verdünnung der Polyurethanharnstoff-Dispersion können gängige Lösemittel, bevorzugt jedoch Wasser eingesetzt werden.

Weiterhin können als Zuschlagstoffe Haftverstärker eingesetzt werden um die Klebrigkeit der Produkte einzustellen. Als Haftverstärker (Tackifier) können die im Stand der Technik bekannten Zuschlagstoffe eingesetzt werden. Beispiele sind: wassermischbare, mono-, di- und multifunktionelle Hydroxyverbindungen, bevorzugt aliphatischer Natur, wie z.B. Glycerin, Ethylenglykol, Propylenglykol, Di-, Tri und Tetraethylenglycol, TMP, besonders bevorzugt Glycerin und Triethylenglykol, kurzkettige Polyethylenoxide wie z.B. PEG 200, PEG 300, PEG400, Rosinester, Copolymere auf Basis von Styrol und Acrylsäureester oder Phenolether oder auch Gemische der genannten Verbindungen.

Weiterhin kann es vorteilhaft sein die Oberflächenklebrigkeit, die zu einem Verblocken der Bandage auf der Rolle führen kann, durch den Zusatz von Füllstoffen als Zuschlagstoffe zu regulieren. Dies können sein: Kieselgel, Silicate, Talkum, Magnesia, Calcit, Harnstoff und - derivate oder andere pulverförmige Feststoffe, insbesondere solche, die sich in die Polyurethanharnstoff-Dispersion homogen einarbeiten lassen. Weiterhin können auch flüssige Additive gegen Verblocken eingesetzt werden, beispielsweise Öl-basierte Systeme, vorzugsweise Silkon-haltige Systeme.

Typische weitere geeignete Zuschlagstoffe sind Oberflächenadditive wie z.B. Benetzungshilfsmittel, Farbstoffe und/oder Verlaufshilfsmittel. Die Polyurethanharnstoffzusammensetzung kann auch alle weiteren dem Fachmann für die jeweilige Anwendung bekannten Zuschlagstoffe enthalten.

Die Auftragung auf das Substrat in Schritt I) kann generell mit allen bekannten Auftragungstechniken erfolgen, speziell mittels Rakel, Tauchbad, Quetschwalze (oder Walzenstuhl, Fachbegriff: squeegee), Drucken oder Sprühauftrag, dabei sind Tauchen, Sprühauftrag und Quetschwalze bevorzugt und besonders bevorzugt Sprühauftrag und Quetschwalze.

Beim Auftrag durch die Quetschwalze wird die Polyurethanharnstoffzusammensetzung bevorzugt auf die Walzen aufgetragen, deren Abstand und Anpressdruck für die Erzielung der gewünschten Schichtstärke optimiert wurde. Dann kann das Textilsubstrat durch die Walzen geführt werden, wobei die Polyurethanharnstoffzusammensetzung im eingestellten Maße auf das Textil aufgetragen wird. Somit erfolgt bevorzugt eine beidseitige Beschichtung. Besonders bevorzugt ist, wenn die Auftragung mit einem bis zwei, besonders bevorzugt in einem einzigen Durchlauf durch die Quetschwalzen erfolgen kann.

Für den Rakelauftrag kann das Substrat in einer Spannvorrichtung zuvor fixiert werden und anschließend kann das Rakel mit der Dispersion davorliegend per Hand oder automatisiert über das Substrat geführt und dabei die Dispersion gleichmäßig auf diesem verteilt werden. Ebenso kann die Beschichtung über eine typische Rolle-zu-Rolle-Beschichtungsanlage mit Rakel erfolgen, bei der das Substrat kontinuierlich beschichtet wird.

Beim Sprühverfahren wird das Substrat bevorzugt in einen Rahmen gespannt und mit der Dispersion aus einer Sprühpistole ein- oder beidseitig besprüht. Der Auftrag kann in einem oder mehreren Kreuzgängen manuell oder über eine kontinuierliche Rolle-zu-Rolle-Sprühanlage erfolgen.

Beim Tauchverfahren durchläuft das Substrat bevorzugt ein Dispersionsbad enthaltend die Polyurethanharnstoffzusammensetzung und wird dadurch mit dieser benetzt. Durch die Verweilzeit im Bad, die Konzentration (bzw. den Feststoffgehalt) der Polyurethanharnstoffzusammensetzung und deren Viskosität können die aufgetragenen Schichtstärken kontrolliert werden. Eine Abtragswalze oder ein Paar Quetschwalzen können verwendet werden, um überschüssige Polyurethanharnstoffzusammensetzung zu entfernen. Bevorzugt erfolgt hier eine beidseitige Beschichtung in einem einzigen Durchlauf.

Nach Auftragung der Polyurethanharnstoffzusammensetzung auf das Substrat, bevorzugt nach einem der oben beschriebenen Verfahren, wird die Beschichtung in Schritt II) getrocknet. Die Trocknung erfolgt durch thermische Trocknung, bei Temperaturen zwischen 20°C und 200°C, bevorzugt zwischen 40°C und 150°C und besonders bevorzugt zwischen 60°C und 120°C. Die thermische Trocknung kann durch IR- oder Mikrowellentrocknung ersetzt oder unterstützt werden.

Bevorzugt wird bei der Auftragung der Polyurethanharnstoffzusammensetzung auf das Substrat mindestens eine Fläche des Substrates an der Oberfläche beschichtet, besonders bevorzugt werden zwei gegenüberliegende Flächen des Substrates (Vorder- und Rückseite) an der Oberfläche beschichtet. Ganz besonders erfolgt die Beschichtung mit der Polyurethanharnstoffzusammensetzung dabei gleichmäßig.

Eine Imprägnierung des Substrates mit der Polyharnstoffzusammensetzung über die vollständige Fläche und Dicke ist möglich. Bevorzugt verbleibt die Polyurethanharnstoffzusammensetzung jedoch an der Oberfläche des Substrates und dringt nicht vollständig in dessen Innenbereich (Bulk) oder die Fasern selbst ein.

Ein weiterer Gegenstand der Erfindung ist ein Polyurethanharnstoff, welcher erhältlich ist, durch Umsetzung von wenigstens
A) einer aliphatischen Polyisocyanat-Komponente mit einer mittleren Isocyanatfunktionalität von ≥ 1,8 und ≤ 2,6,
B) einer polymeren Polyether-Polyol-Komponente, welche ein Gemisch aus Poly(tetramethylenglykol)polyetherpolyolen enthält oder daraus besteht, wobei sich die Poly(tetramethylenglykol)polyetherpolyole in ihren zahlenmittleren Molekulargewichten unterscheiden,
C) einer aminofunktionellen Kettenverlängerer-Komponente mit mindestens 2 isocyanatreaktiven Aminogruppen, enthaltend wenigstens eine aminofunktionelle Verbindung C1), die keine ionischen oder ionogenen Gruppen aufweist und eine aminofunktionelle Verbindung C2), die ionische oder ionogene Gruppen aufweist,
D) gegebenenfalls weiteren hydrophilierenden Komponenten, die unterschiedlich sind von C2),
E) gegebenenfalls hydroxyfunktionellen Verbindungen mit einem Molekulargewicht von 62 bis 399 mol/g,
F) gegebenenfalls weitere polymere Polyolen, welche unterschiedlich sind von B)
G) einer Verbindung, die genau eine isocyanatreaktive Gruppe aufweist, oder einer Verbindung, die mehr als eine isocyanatreaktive Gruppe aufweist, wobei nur eine der isocyanat reaktiven Gruppen unter den gewählten Umsetzungsbedingungen mit den in der Reaktionsmischung vorhandenen Isocyanatgruppen reagiert und
H) einer aliphatischen Polyisocyanat-Komponente mit einer mittleren Isocyanatfunktionalität von > 2,6 und ≤ 4,
wobei die Komponenten B) und F) zusammen ≤ 30 Gew.-% an Komponente F), bezogen auf die Gesamtmasse der Komponenten B) und F) enthalten und die Komponenten G) und H) in einem molaren Verhältnis von 5:1 bis 1:5 zueinander vorliegen.

Erfindungsgemäß beträgt das molare Verhältnis von Komponente G) zu Komponente H) 5:1 bis 1:5, besonders bevorzugt 1,5:1 bis 1:4 und ganz besonders bevorzugt 1:1 bis 1:3.

Für die Komponenten A) bis H) gelten hier analog die für den im erfindungsgemäßen Produkt enthaltenen Polyurethanharnstoff oben genannten Definitionen und bevorzugten Ausführungsformen.

Insbesondere bevorzugt ist die Komponente A) Isophorondiisocyanat und/oder Hexamethylendiisocyanat.

Erfindungsgemäß ist die Komponente B) ein Gemisch aus Poly(tetramethylenglykol)polyetherpolyolen enthält oder daraus besteht, wobei sich die Poly(tetramethylenglykol)polyetherpolyole in ihren zahlenmittleren Molekulargewichten unterscheiden.

Erfindungsgemäß ist die Komponente C) wenigstens eine aminofunktionelle Verbindung C1), die keine ionischen oder ionogenen Gruppen aufweist und eine aminofunktionelle Verbindung C2), die ionische oder ionogene Gruppen aufweist enthält.

Ebenfalls insbesondere bevorzugt handelt es sich bei der Komponente D) um nichtionisch hydrophilierende Komponenten.

Besonders vorteilhafte Ausführungsformen der Erfindung ergeben sich auch durch die Kombinationen der hier vorstehend als insbesondere bevorzugt genannten Merkmale.

In einer bevorzugten Ausführungsform werden zur Herstellung der erfindungsgemäß verwendeten Polyurethanharnstoffe die Komponenten A) bis H) in den folgenden Mengen eingesetzt, wobei sich die Einzelmengen stets zu 100 Gew.-% addieren:
5 bis 40 Gew.-% Komponente A),
55 bis 90 Gew.-% Summe der Komponenten B) und gegebenenfalls F),
0,5 bis 20 Gew.-% Summe der Komponenten C1) und gegebenenfalls E),
0,1 bis 10 Gew.-% Komponente C2),
0 bis 20 Gew.-% Komponente D),
0,1 bis 20 Gew.-% der Komponente G) und
0 bis 10 Gew.-% Komponente H).

In einer weiteren bevorzugten Ausführungsform werden zur Herstellung der erfindungsgemäß verwendeten Polyurethanharnstoffe die Komponenten A) bis H) in den folgenden Mengen eingesetzt, wobei sich die Einzelmengen stets zu 100 Gew.-% aufaddieren:
10 bis 35 Gew.-% Komponente A),
60 bis 85 Gew.-% Summe der Komponenten B) und gegebenenfalls F),
1 bis 15 Gew.-% Summe der Komponenten C1) und gegebenenfalls E),
0,5 bis 4 Gew.-% Komponente C2),
0 bis 10 Gew.-% Komponente D),
0,3 bis 10 Gew.-% der Komponente G) und
0,1 bis 3 Gew.-% Komponente H).

In einer besonders bevorzugten Ausführungsform der Erfindung umfasst das kontaktklebende Produkt einen Polyurethanharnstoff, der erhältlich ist, durch Umsetzung von wenigstens
A) einer aliphatischen Polyisocyanat-Komponente, wobei es sich um ein Gemisch aus IPDI und HDI handelt,
B) einer polymeren Polyether-Polyol-Komponente, welche ein Gemisch aus wenigstens zwei Poly(tetramethylenglykol)polyetherpolyolen ist und wobei sich die Poly(tetramethylenglykol)polyetherpolyole in ihren zahlenmittleren Molekulargewichten unterscheiden,
C) einer aminofunktionellen Kettenverlängerer-Komponente mit 2 isocyanatreaktiven primären und/oder sekundären Aminogruppen, enthaltend wenigstens eine aminofunktionelle Verbindung C1), die keine ionischen oder ionogenen Gruppen aufweist und/oder eine aminofunktionelle Verbindung C2), die ionische oder ionogene Gruppen aufweist,
D) gegebenenfalls weiteren hydrophilierenden Komponenten, die unterschiedlich sind von C2), wobei es sich um nichtionisch hydrophilierende Komponenten D1) handelt,
E) gegebenenfalls hydroxyfunktionellen Verbindungen mit einem Molekulargewicht von 62 bis 399 mol/g,
F) gegebenenfalls weiteren polymeren Polyolen, welche unterschiedlich sind von B),
G) einer Verbindung, die genau eine isocyanatreaktive Gruppe aufweist, oder einer Verbindung, die mehr als eine isocyanatreaktive Gruppe aufweist, wobei nur eine der isocyanat reaktiven Gruppen unter den gewählten Umsetzungsbedingungen mit den in der Reaktionsmischung vorhandenen Isocyanatgruppen reagiert, wobei es sich bei der isocyanat reaktiven Gruppe um eine primäre und/oder sekundäre Amino- und/oder Hydroxygruppe handelt und
H) einer aliphatischen Polyisocyanat-Komponente mit einer mittleren Isocyanatfunktionalität von > 2,6 und ≤ 4, wobei die Komponente H) aus einem aliphatischen oder cycloaliphatischen Polyisocyanat-Oligomer mit Isocyanurat-, Urethan-, Allophanat-, Biuret-, Iminooxadiazindion- oder Oxadiazintrionstruktur, basierend auf HDI, IPDI und/oder H12-MDI besteht,
wobei die Komponenten B) und F) zusammen ≤ 30 Gew.-% an Komponente F), bezogen auf die Gesamtmasse der Komponenten B) und F) enthalten. und die Komponenten G) und H) in einem molaren Verhältnis von 1:1 bis 1:3 zueinander vorliegen.

Ganz besonders bevorzugt ist der erfindungsgemäße Polyurethanharnstoff erhältlich durch Umsetzung ausschließlich der Komponenten A) bis H). Es werden dann keine weiteren Komponenten zur Herstellung des Polyurethanharnstoffs eingesetzt.

Die erfindungsgemäßen Polyurethanharnstoffe sind bevorzugt lineare Moleküle, können alternativ jedoch auch verzweigt sein.

Das zahlenmittlere Molekulargewicht der erfindungsgemäß bevorzugt verwendeten Polyurethanharnstoffe beträgt bevorzugt von ≥ 2000 bis ≤ 300000 g/mol, bevorzugt von ≥ 5000 bis ≤ 150000 g/mol.

Der erfindungsgemäße Polyurethanharnstoff ist bevorzugt amorph und weist einen T_{g} von ≤ - 25 °C, oder bevorzugt von ≤ - 50 °C, oder bevorzugt von ≤ - 70 °C auf.

Der erfindungsgemäße Polyurethanharnstoff liegt bevorzugt in einem physiologisch akzeptablen Medium vor. Besonders bevorzugt handelt es sich bei dem Medium um Wasser und ganz besonders bevorzugt liegt der Polyurethanharnstoff als wässrige Dispersion vor. Wasser bildet im Allgemeinen, neben wahlweise vorhandenen anderen flüssigen Medien, wie beispielsweise Lösungsmitteln, den Hauptbestandteil (> 50 Gew.-%) des Dispergiermediums, bezogen auf die Gesamtmenge des flüssigen Dispergiermediums, gegebenenfalls auch das alleinige flüssige Dispergiermedium.

Die erhaltenen wässrigen Polyurethanharnstoffdispersionen weisen bevorzugt einen Gehalt an flüchtigen organischen Verbindungen (VOC), wie beispielsweise flüchtige organische Lösemitteln, von weniger als 10 Gew.-%, bevorzugter von weniger als 3 Gew.-%, noch bevorzugter von weniger als 1 Gew.-% bezogen auf die auf wässrige Polyurethanharnstoffdispersion auf. VOCs im Sinne dieser Erfindung sind insbesondere organische Verbindung mit einem Anfangssiedepunkt von höchstens 250 °C bei einem Standarddruck von 101,3 kPa.

Die Bestimmung des Gehalts an flüchtigen organischen Verbindungen (VOC) erfolgt im Rahmen der vorliegenden Erfindung insbesondere durch gaschromatographische Analyse.

Der pH-Wert der wässrigen Polyurethan-Dispersionen beträgt typischerweise weniger als 8,0, bevorzugt weniger als 7,5 und liegt besonders bevorzugt zwischen 5,5 und 7,5.

Um eine gute Sedimentationsstabilität zu erreichen, liegt die zahlenmittlere Teilchengröße der speziellen Polyurethanharnstoff-Dispersionen bevorzugt bei weniger als 750 nm, besonders bevorzugt bei weniger als 500 nm, bestimmt mittels Laserkorrelations-Spektroskopie nach Verdünnung mit entionisiertem Wasser (Gerät: Malvern Zetasizer 1000, Malvern Inst. Limited).

Der Feststoffgehalt der Polyurethanharnstoff-Dispersionen beträgt bevorzugt 10 bis 70 Gew.-%, besonders bevorzugt 20 bis 60 Gew.-%und ganz besonders bevorzugt 40 bis 60 Gew.-%. Die Feststoffgehalte werden ermittelt durch Erhitzen einer ausgewogenen Probe auf 125°C bis zur Gewichtskonstanz. Bei Gewichtskonstanz wird durch erneutes Auswiegen der Probe der Festkörpergehalt berechnet.

Bevorzugt weisen diese Polyurethanharnstoff-Dispersionen weniger als 5 Gew.-%, besonders bevorzugt weniger als 0,2 Gew.-%, bezogen auf die Masse der Dispersionen, an ungebundenen organischen Aminen auf.

Die Polyurethanharnstoff-Dispersion weist bei einer konstanten Scherrate von 10 s⁻¹ bevorzugt eine Viskosität von ≥ 1 und ≤ 10000 mPa s, besonders bevorzugt von ≥ 10 und ≤ 5000 mPa s und ganz besonders bevorzugt von ≥ 100 und ≤ 4000 mPa s auf. Die Viskosität wird dabei wie im Methodenteil beschrieben bestimmt.

Bevorzugt ist der verwendete Polyurethanharnstoff daher in Wasser dispergierbar, was im Rahmen dieser Erfindung bedeutet, dass der Polyurethanharnstoff bei 23°C eine sedimentationsstabile Dispersion in Wasser, insbesondere entionisiertem Wasser, bilden kann.

Der erfindungsgemäße Polyurethanharnstoffe ist bevorzugt erhältlich, indem isocyanatfunktionelle Polyurethanpräpolymere a) aus den Komponenten A), H), B) und gegebenenfalls D) und/oder C2), sowie gegebenenfalls den Verbindungen E) hergestellt werden (Schritt a) und deren freie NCO-Gruppen anschließend ganz oder teilweise mit der aminofunktionellen Kettenverlängerer-Komponente C), sowie der Komponente G) und gegebenenfalls der Komponente D) umgesetzt werden (Schritt b)). Die Komponente H) kann auch erst in Schritt b) eingesetzt werden, was jedoch weniger bevorzugt ist. Wenn die Komponente H) erst in Schritt b) eingesetzt wird, wird diese bevorzugt vor der Zugabe der Komponente C) zugegeben und mit dem Prepolymer a) umgesetzt.

Die Komponenten A) bis H) sind dabei wie oben angegeben definiert und es gelten auch für das Herstellungsverfahren die oben genannten Ausführungsformen inklusive aller Vorzugsbereiche.

Gegenstand der Erfindung ist daher ebenfalls ein Verfahren zur Herstellung des erfindungsgemäßen Polyurethanharnstoffs, bei dem isocyanatfunktionelle Polyurethanpräpolymere a) aus den Komponenten A), H), B) und gegebenenfalls D) und/oder C2), sowie gegebenenfalls den Verbindungen E) hergestellt werden (Schritt a) und deren freie NCO-Gruppen anschließend ganz oder teilweise mit der aminofunktionellen Kettenverlängerer-Komponente C), sowie der Komponente G) und gegebenenfalls der Komponente D) umgesetzt werden (Schritt b)). Die Komponente H) kann auch erst in Schritt b) eingesetzt werden, was jedoch weniger bevorzugt ist. Wenn die Komponente H) erst in Schritt b) eingesetzt wird, wird diese bevorzugt vor der Zugabe der Komponente C) zugegeben und mit dem Prepolymer a) umgesetzt.

Die Komponenten A) bis H) sind dabei ebenfalls wie oben angegeben definiert und es gelten auch für das Herstellungsverfahren die oben genannten Ausführungsformen inklusive aller Vorzugsbereiche.

In einer bevorzugten Ausführungsform werden die Komponenten A) und H) schon vorgemischt in Schritt a) eingesetzt.

Ebenfalls Gegenstand der Erfindung ist ein Klebstoff, bevorzugt ein Kontaktklebstoff, umfassend den erfindungsgemäßen Polyurethanharnstoff.

Weiterhin ist Gegenstand der Erfindung ein Objekt, hergestellt durch Verkleben von zwei oder mehr Substraten mittels des erfindungsgemäßen Polyurethanharnstoff oder des erfindungsgemäßen Klebstoffes.

Als Substrate eignen sich bevorzugt textile Flächengebilde, Flächensubstrate aus Metall, Glas, Keramik, Beton, Naturstein, Leder, Naturfasern, und Kunststoffen wie PVC, Polyolefine, Polyurethan oder ähnliches. Auch dreidimensionale Gebilde eigenen sich als Substrate. Besonders bevorzugt sind die oben für das kontaktklebende Produkt näher aufgeführten Substrate.

Ein erfindungsgemäßes Objekt kann sein ein Fortbewegungsmittel wie ein Auto, ein Motorrad, ein Flugzeug, ein Zug; ein LKW oder ein Fahrrad; ein Elektroartikel wie ein Mobiltelefon oder ein Computer; ein Gebäude; ein Möbelstück; ein Förderband, eine Baumaschine, Verpackungsmaterial, Werkzeug, ein Bürogegenstände, ein Kleidungsstück, ein Schuh, ein Haushaltsartikel, ein Medizintechik-Artikel ;wobei sich die Klebung auch auf einzelne oder mehrere Teile der genannten Objekte beziehen kann.

Weiterhin ist ein Gegenstand der Erfindung eine wässrige Dispersion enthaltend einen erfindungsgemäßen Polyurethanharnstoff.

Weiterhin ist ein Gegenstand der Erfindung die Verwendung des erfindungsgemäßen Polyurethanharnstoffs oder Kontaktklebstoffes zur Herstellung von kontaktklebrigen Substraten wie beispielsweise Klebebändern für den Gebrauch im Haushalt oder Handwerk, sowie für den industriellen Gebrauch.

Weiterhin ist ein Gegenstand der Erfindung die Verwendung des erfindungsgemäßen Polyurethanharnstoffs oder Kontaktklebstoffes zur Herstellung von Pflastern, Verbänden, Tapes oder Bandagen.

Die vorliegende Erfindung wird an Hand der folgenden Beispiele erläutert.

### Beispiele:

### Methoden:

Sofern nicht abweichend gekennzeichnet, beziehen sich alle Prozentangaben auf das Gewicht und die Gesamtmenge bzw. auf das Gesamtgewicht der Zusammensetzungen.

Sofern nicht abweichend vermerkt, beziehen sich alle analytischen Messungen auf Messungen bei Temperaturen von 23 °C.

Die Feststoff- bzw. Festkörpergehalte wurden entsprechend DIN EN ISO 3251 durch Erhitzen einer ausgewogenen Probe auf 105 °C bis zur Gewichtskonstanz ermittelt. Bei Gewichtskonstanz wurde durch erneutes Auswiegen der Probe der Festkörpergehalt berechnet.

NCO-Werte wurden, wenn nicht ausdrücklich anders erwähnt, volumetrisch gemäß DIN-EN ISO 11909 bestimmt.

Die Kontrolle auf freie NCO-Gruppen wurde mittels IR-Spektroskopie (Bande bei 2260 cm⁻¹) durchgeführt.

Die angegebenen Viskositäten wurden mittels Rotationsviskosimetrie nach DIN 53019 bei 23 °C mit einem Rotationsviskosimeter der Firma Anton Paar Germany GmbH, Ostfildern, DE bestimmt (1 Pa s = 1 N/m²*s).

Die Bestimmung der mittleren Teilchengrößen (angegeben ist das Zahlenmittel) der Polyurethan-Dispersionen erfolgte nach Verdünnen mit entionisiertem Wasser mittels Laserkorrelations-Spektroskopie (Gerät: Malvern Zetasizer 1000, Malver Inst. Limited).

Der pH-Wert wurde gemäß der in DIN ISO 976 beschriebenen Methode an der unverdünnten Probe gemessen.

Die Glasübergangstemperatur T_{g} wurde mittels dynamischer Differenzkalorimetrie (DSC) in Anlehnung an die DIN EN 61006, Verfahren A, bestimmt, wobei ein DSC Gerät (Kalorimeter Pyris Diamond DSC von Perkin-Elmer) verwendet wird, das zur Bestimmung von T_{g} mit Indium und Blei kalibriert ist. Es werden 10 mg der zu untersuchenden Substanz in einen verschließbaren Aluminium-Tiegel eingewogen und dieser verschlossen. Es werden drei unmittelbar aufeinander folgende Durchläufe einer Aufheizungen von -100°C bis +150°C, bei einer Heizrate von 20 K/min, mit anschließender Abkühlung mit einer Kühlrate von 320 K/min vorgenommen und die dritte Aufheizkurve zur Bestimmung der Werte verwendet. Als T_{g} wird die Temperatur bei der halben Höhe einer Glasübergangsstufe bestimmt.

Die Bestimmung der Luftdurchlässigkeit der Substrate im ungedehnten Zustand erfolgte gemäß DIN EN ISO 9237.

Die Bestimmung der Höchstzugkraft der Substrate erfolgte mit einem Streifen - Zugversuch gemäß DIN EN ISO 13934-1.

Die Bestimmung des Dehnungsbereichs der Substrate erfolgte durch Bestimmung des zugelastischen Verhaltens der Substrate mittels mehrmaliger Zugbeanspruchung zwischen konstanten Dehngrenzen sowie die Bestimmung der Gesamtdehnung gemäß DIN 53835 Teil 2.

### Verwendete Substanzen und Abkürzungen:

- Diaminosulfonat:: NH₂-CH₂CH₂-NH-CH₂CH₂-SO₃Na (45 %ig in Wasser)
- PolyTHF 1000: Poly(tetramethylenglykol)polyetherdiol mit der zahlenmittleren Molmasse von 1000 g/mol, BASF SE, Ludwigshafen, DE
- PolyTHF 2000: Poly(tetramethylenglykol)polyetherdiol mit der zahlenmittleren Molmasse von 2000 g/mol, BASF SE, Ludwigshafen, DE
- Wasser: Durch Ionenaustauscher vollentsalztes Wasser

Die eingesetzten Isocyanate-Komponenten sind Handelsprodukte der Covestro Deutschland AG, Leverkusen, DE. Weitere Chemikalien von Sigma-Aldrich Chemie GmbH, Taufkirchen, DE. Die Rohstoffe wurden, soweit nicht anders erwähnt, ohne weitere Reinigung oder Vorbehandlung eingesetzt.

### Nicht erfindungsgemäße Polyurethanharnstoff-Dispersion 1

360 g PolyTHF^{®} 1000 und 1680 g PolyTHF^{®} 2000 wurden auf 70°C aufgeheizt. Anschließend wurde ein Gemisch aus 180,6 g Hexamethylendiisocyanat und 238,7 g Isophorondiisocyanat zugegeben und solange bei 100-115°C gerührt bis der theoretische NCO-Wert unterschritten war. Das fertige Präpolymer wurde mit 4400 g Aceton bei 50°C gelöst und anschließend eine Lösung aus 19,6 g Ethylendiamin, 86,3 g Diaminosulfonat, 27,9 g Diethanolamin und 380 g Wasser zudosiert. Die Nachrührzeit betrug 15 min. Danach wurde durch Zugabe von 2100 g Wasser dispergiert. Es folgte die Entfernung des Lösemittels durch Destillation im Vakuum und es wurde eine lagerstabile Dispersion erhalten; der Festgehalt wurde durch Zusatz von Wasser eingestellt.

| | |
|---|---|
| Feststoffgehalt: | 52 % |
| Partikelgröße (LKS): | 292 nm |
| Viskosität: | 440 mPa s |
| Tg Polyurethanharnstoff: | -78,7 °C |

### Erfindungsgemäße Polyurethanharnstoff-Dispersion 2

75 g PolyTHF^{®} 1000 und 350 g PolyTHF^{®} 2000 wurden auf 70°C aufgeheizt. Anschließend wurde ein Gemisch aus 33,9 g Hexamethylendiisocyanat , 49,7 g Isophorondiisocyanat, sowie 8,7 g Desmodur N 3300 (HDI-Trimerisat mit einem NCO-Gehalt von ca. 21,8% nach DIN EN ISO 11 909) zugegeben und solange bei 100-115°C gerührt bis der theoretische NCO-Wert unterschritten war. Das fertige Präpolymer wurde mit 920 g Aceton bei 50°C gelöst und anschließend eine Lösung aus 3,2 g Ethylendiamin, 12,9 g Diaminosulfonat, 11,7 g Diethanolamin und 145 g Wasser zudosiert. Die Nachrührzeit betrug 15 min. Danach wurde durch Zugabe von 1080 g Wasser dispergiert. Es folgte die Entfernung des Lösemittels durch Destillation im Vakuum und es wurde eine lagerstabile Dispersion erhalten; der Festgehalt wurde durch Zusatz von Wasser eingestellt.

| | |
|---|---|
| Feststoffgehalt: | 52 % |
| Partikelgröße (LKS): | 307 nm |
| Viskosität: | 105 mPa s |
| Tg Polyurethanharnstoff: | -78,0 °C |

### Polyurethanharnstoffdispersion V1 (Vergleich 1)

450 g PolyTHF^{®} 1000 und 2100 g PolyTHF^{®} 2000 wurden auf 70°C aufgeheizt. Anschließend wurde ein Gemisch aus 225,8 g Hexamethylendiisocyanat und 298,4 g Isophorondiisocyanat zugegeben und solange bei 100-115°C gerührt bis der theoretische NCO-Wert unterschritten war. Das fertige Prepolymer wurde mit 5460 g Aceton bei 50°C gelöst und anschließend eine Lösung aus 29,5 g Ethylendiamin, 143,2 g Diaminosulfonat und 610 g Wasser zudosiert. Die Nachrührzeit betrug 15 min. Danach wurde durch Zugabe von 1880 g Wasser dispergiert. Es folgte die Entfernung des Lösemittels durch Destillation im Vakuum und es wurde eine lagerstabile Dispersion erhalten, der Festgehalt wurde durch Zusatz von Wasser eingestellt.

| | |
|---|---|
| Feststoffgehalt: | 56 % |
| Partikelgröße (LKS): | 276 nm |
| Viskosität: | 1000 mPas |
| Tg Polyurethanharnstoff: | -79,1 °C |

### Polyurethandispersion V2 gemäß US 5,692,937 (Vergleich 2)

Es wurde die in der US 5,692,937 Beispiel 1 (Spalte 4, Zeile 15 bis 34) beschriebene Polyurethandispersion nachgestellt. Hierbei wurde das IPDI sowie die Polyole von Covestro AG, Leverkusen, DE verwendet, während alle anderen Chemikalien von Sigma-Aldrich Chemie GmbH, Taufkirchen, DE bezogen wurden.

Die Polyurethandispersionen der Vergleichsbeispiele wurden in Apparaturen und unter Bedingungen hergestellt, die denen der in US 5,692,937 und den erfindungsgemäßen Beispiele vergleichbar sind.

### Anwendungsversuche:

### Verwendete Materialien:

Ypsiflex-Binde der Firma Holthaus Ref. 12906S: Luftdurchlässigkeit im ungedehnten Zustand: 5548 l/m2*s, Dehnungsbereich: 160%, Höchstzugkraft: 155,9 N;
Ypsifix-Binde der Firma Holthaus Ref. 12223

### Prüfung der Kontaktklebrigkeit:

Nach der Auftragung und Trocknung der Polyurethanharnstoffzusammensetzung auf eine 30cm lange Ypsifix-Binde (Ref.12223) oder Ypsiflex-Binde (Ref.12906S), wird diese um einen Stift gewickelt, so dass die Wicklungen übereinander liegen. Nach 14 Tagen wird die Kontaktklebkraft des Produkts mit sich selbst überprüft. Dies erfolgt indem zwei 3cm lange Stücke übereinander gelegt, leicht mit den Fingern für 10 s bei Raumtemperatur zusammengedrückt und darauffolgend direkt das Ablöseverhalten durch auseinanderziehen der beiden Stücke optisch ermittelt wird. Die Skala der Beurteilung reicht von 1 (klebt nicht aneinander) bis 5 (klebt sehr stark aneinander). Ab einer Einstufung von "3" gilt die Kontaktklebrigkeit als ausreichend.

### Anwendungsbeispiel A1 (nicht erfindungsgemäß):

97g der nicht erfindungsgemäßen Polyurethanharnstoffdispersion 1 wurden mit 3g Glycerin in einem Speedmixer-Becher vorgelegt. Die blasenfreie Vermischung zu einer Polyurethanharnstoffzusammensetzung erfolgte in dem Speedmixer bei einer Umdrehungszahl von 2750 min⁻¹ für 1 Minute. Für den folgenden Sprühversuch wurde die zu benetzende Holthaus Ypsiflex - Mullbinde (Ref.12906S) (6x30 cm²) (Substrat) in einem starren Rahmen fixiert. Die Formulierung wurde aus dem Speedmixer-Becher in die Vorlage deiner Sprühpistole (SATA Jet RP Digital) überführt. Mittels eines Luftdrucks von 1,5 bar wurde die Polyurethanharnstoffzusammensetzung über eine Düse (Durchmesser 1,6 mm) auf das Substrat tropfenförmig verteilt. Das Substrat wurde von jeder Seite einmal besprüht. Vor der Trocknung für 10 min bei 100 °C in einem Umlufttrockenschrank erfolgte eine Vortrocknung für 20min bei RT. Nach der Trocknung wird die 30cm lange beschichtete Bandage um einen Stift gewickelt, so dass die Wicklungen übereinander lagen. Die beschichtete Binde haftete in der Aufwicklung nur leicht aneinander und verklebte auch nach 14 Tagen Lagerung nicht. Die Beurteilung der Kontaktklebrigkeit erfolgt nach 14 Tagen und ist in Tabelle 1 aufgeführt.

### Anwendungsbeispiel A2 (Vergleich):

97g der Vergleichs-Polyurethanharnstoffdispersion V1 wurden mit 3g Glycerin in einem Speedmixer-Becher vorgelegt. Die blasenfreie Vermischung zu einer Polyurethanharnstoffzusammensetzung erfolgte in dem Speedmixer bei einer Umdrehungszahl von 2750 min⁻¹ für 1Minute. Für den Sprühversuch wurde die zu benetzende Holthaus Ypsiflex - Mullbinde (Ref.12906S) (6x30 cm²) (Substrat) in einem starren Rahmen fixiert. Die Formulierung wurde aus dem Speedmixer-Becher in die Vorlage einer Sprühpistole (SATA Jet RP Digital) überführt. Mittels eines Luftdrucks von 1,5 bar wurde die Polyurethanharnstoffzusammensetzung über eine Düse (Durchmesser 1,6 mm) auf das Substrat tropfenförmig verteilt. Das Substrat wurde von jeder Seite einmal besprüht. Vor der Trocknung für 10 min bei 100 °C in einem Umlufttrockenschrank erfolgte eine Vortrocknung für 20 min bei RT. Nach der Trocknung wurde die 30 cm lange beschichtete Bandage um einen Stift gewickelt, so dass die Wicklungen übereinander lagen. Die Beurteilung der Kontaktklebrigkeit erfolgt nach 14 Tagen und ist in Tabelle 1 aufgeführt.

### Anwendungsbeispiel A3 (erfindungsgemäß):

Für den Sprühversuch wurde die zu benetzende Ypsifix - Mullbinde (Ref.12223) (6x30 cm²) (Substrat) in einen starren Rahmen fixiert. 100g der unbehandelten, erfindungsgemäßen Polyurethanharnstoffdispersion 2 wurden in die Vorlage einer Sprühpistole (SATA Jet RP Digital) überführt. Mittels eines Luftdrucks von 1,5 bar wurde die Dispersion über eine Düse (Durchmesser 1,6 mm) auf das Substrat tropfenförmig verteilt. Das Substrat wurde von jeder Seite einmal besprüht. Die Trocknung erfolgte für 60 min bei 100°C in einem Umlufttrockenschrank. Nach der Trocknung wurde die 30cm lange beschichtete Bandage um einen Stift gewickelt, so dass die Wicklungen übereinander lagen. Die beschichtete Binde haftete in der Aufwicklung nur leicht aneinander und verklebte auch nach 14 Tagen Lagerung nicht. Die Beurteilung der Kontaktklebrigkeit erfolgt nach 14 Tagen und ist in Tabelle 1 aufgeführt.

### Anwendungsbeispiel A4 (nicht erfindungsgemäß):

180g der nicht erfindungsgemäßen Polyurethanharnstoffdispersion 1 wurden mit 0,9 g Rheolate 678 mittels eines KPG-Laborrührwerk (Umdrehungsgeschwindigkeit: 1100min⁻¹ und Rührdauer: 5min) zu einer Polyurethanharnstoffzusammensetzung vermengt. Die zu benetzende Ypsiflex-Binde (Ref.12906S) (6x30 cm²) (Substrat) wurde in einen Spannrahmen eines Ofens der Firma Mathis fixiert. Die hergestellte Polyurethanharnstoffzusammensetzung wurde im oberen Teil des fixierten Textils aufgegeben und anschließend durch Überziehen mit einem Rakel gleichmäßig verteilt. Der Rakelspalt betrug 100µm. Die Trocknung erfolgte im Ofen der Firma Mathis bei 120 °C für 2 min. Die Kontaktklebrigkeit der nichtaufgerollten Binde wurde nach 14 Tagen geprüft und ist in Tabelle 1 aufgeführt.

### Anwendungsbeispiel A5 (Vergleich):

Für den Sprühversuch wurde die zu benetzende Ypsiflex-Mullbinde (Ref.12906S) (6x30 cm²) (Substrat) in einen starren Rahmen fixiert. 100 g der unbehandelten Vergleichs-Polyurethanharnstoffdispersion V2 wurden in die Vorlage einer Sprühpistole (SATA Jet RP Digital) überführt. Mittels eines Luftdrucks von 1,5 bar wurde die Dispersion über eine Düse (Durchmesser 1,6 mm) auf das Substrat tropfenförmig verteilt. Das Substrat wurde von jeder Seite einmal besprüht. Vor der Trocknung für 10 min bei 100 °C in einem Umlufttrockenschrank erfolgte eine Vortrocknung für 20 min bei RT. Nach der Trocknung wurde die 30 cm lange, beschichtete Bandage um einen Stift gewickelt, so dass die Wicklungen übereinander lagen. Die Beurteilung der Kontaktklebrigkeit erfolgt nach 14 Tagen und ist in Tabelle 1 aufgeführt.

**Tabelle 1: Prüfung der Kontaktklebrigkeit nach 14 Tagen:**

| Nach 14 Tagen | Beispiel A1 (nicht erfindungsgemäß) | Beispiel A2 (Vergleich) | Beispiel A3 | Beispiel A4 (nicht erfindungsgemäß) | Beispiel A5 (Vergleich) |
|---|---|---|---|---|---|
| Bewertung | 3 | 1 | 4 | 3 | 1-2 |

### Prüfung der Verfärbung:

Die Bestimmung der Farbwerte (L, a und b gemäß CIELab-System) erfolgt von Filmen mit 100 µm Schichtdicke, die auf 20 cm * 10 cm großen und 3 mm dicken Glasplatten der Firma Glas & Fenster Engelbrecht GmbH, Leichlingen (Rheinland), Deutschland, hergestellt wurden (Auftragung mit einem Kastenrakel der Spaltbreite 200 µm und anschließende Trocknung). Die Schichtdickenbestimmung wurde mit einem Drucklufttaster und zur Ausgabe der Schichtdicke angeschlossenem Display der Firma Heidehain (MT25P) ermittelt. Allen Formulierungen wurde zur besseren Filmbildung ein Verdicker zugesetzt (0,5 Gew.-% auf 100 Gew.-% Dispersion).

Die Farbwertmessung erfolgte in Transmission mit dem Gerät CM5 der Firma Konica Minolta, die Farbwertberechnung erfolgte nach DIN11664-4 und die Messgeometrie wurde nach DIN5033-7 mit den Parametern d/8, D65 und SCi festgelegt. Der b-Wert beschreibt die Gelbfärbung eines Films. In Tabelle 2 sind Messergebnisse aufgeführt. Die Messung wurde ca. 10 Tage nach Herstellung der Filme durchgeführt. Die Abweichung der Dicke der Filme betrug lediglich maximal 1 %, bezogen auf die dickste Stelle des Films.

Es ist zu erkennen, dass die b-Werte der erfindungsgemäßen Proben eine deutlich niedrigere Verfärbung gegenüber den Vergleichsproben aufweisen. Insbesondere die aliphatischen Isocyanate sind anders als aromatische Isocyanate nicht lichtempfindlich. Während aromatische Isocyanate beim Aussetzen am Sonnenlicht eine gelb Verfärbung zeigen, zeigen aliphatische Isocyanate keinerlei Verfärbung durch UV-Strahlung wie dem Sonnenlicht.

Für die Farbmessung verwendete Formulierungen:

### Anwendungsbeispiel A6 (nicht erfindungsgemäß), entspricht A1 und A4:

100 g der nicht erfindungsgemäßen Polyurethanharnstoffdispersion 1 wurden mit 3 g Glycerin und 0,5 g Rheolate 210 in einem Speedmixer-Becher vorgelegt. Die blasenfreie Vermischung zu einer Polyurethanharnstoffzusammensetzung erfolgte in dem Speedmixer bei einer Umdrehungszahl von 2750 min⁻¹ für 1 Minute. Nach der Applizierung mittels Rakel und vor der Trocknung für 10 min bei 50 °C und 3 min bei 120 °C in einem Umlufttrockenschrank erfolgte eine Vortrocknung für 20 min bei RT.

### Anwendungsbeispiel A7 (Vergleich), entspricht A2:

100 g der Vergleichs-Polyurethanharnstoffdispersion V1 wurden mit 3 g Glycerin und 0,5 g Rheolate 210 in einem Speedmixer-Becher vorgelegt. Die blasenfreie Vermischung zu einer Polyurethanharnstoffzusammensetzung erfolgte in dem Speedmixer bei einer Umdrehungszahl von 2750 min⁻¹ für 1 Minute. Nach der Applizierung mittels Rakel und vor der Trocknung für 10 min bei 50 °C und 3 min bei 120 °C in einem Umlufttrockenschrank erfolgte eine Vortrocknung für 20 min bei RT.

### Anwendungsbeispiel A8 (erfindungsgemäß), entspricht A3:

100 g der erfindungsgemäßen Polyurethanharnstoffdispersion 2 wurden mit 0,5 g Rheolate 210 in einem Speedmixer-Becher vorgelegt. Die blasenfreie Vermischung zu einer Polyurethanharnstoffzusammensetzung erfolgte in dem Speedmixer bei einer Umdrehungszahl von 2750 min⁻¹ für 1 Minute. Nach der Applizierung mittels Rakel und vor der Trocknung für 10 min bei 50 °C und 3 min bei 120 °C in einem Umlufttrockenschrank erfolgte eine Vortrocknung für 20 min bei RT.

### Anwendungsbeispiel A9 (Vergleich), entspricht A5:

100 g der Vergleichs-Polyurethanharnstoffdispersion V2 wurden mit 0,5 g Rheolate 210 in einem Speedmixer-Becher vorgelegt. Die blasenfreie Vermischung zu einer Polyurethanharnstoffzusammensetzung erfolgte in dem Speedmixer bei einer Umdrehungszahl von 2750 min⁻¹ für 1 Minute. Nach der Applizierung mittels Rakel und vor der Trocknung für 10 min bei 50 °C und 3 min bei 120°C in einem Umlufttrockenschrank erfolgte eine Vortrocknung für 20 min bei RT.

**Tabelle 2: Messung der Farbwerte:**

| | Beispiel A6 (nicht erfindungsgemäß) | Beispiel A7 (Vergleich) | Beispiel A8 | Beispiel A9 (Vergleich) |
|---|---|---|---|---|
| Schichtdicke (µm) | 73 | 98 | 87 | 83 |
| L-Wert | 96,6 | 96,6 | 96,6 | 96,4 |
| a-Wert | -0,5 | -0,5 | -0,5 | -0,6 |
| **b-Wert** | **0,3** | **0,2** | **0,3** | **1,0** |

## Patentansprüche

1. Kontaktklebendes Produkt, umfassend ein Substrat und einen Polyurethanharnstoff, welcher erhältlich ist, durch Umsetzung von wenigstens
A) einer aliphatischen Polyisocyanat-Komponente mit einer mittleren Isocyanatfunktionalität von ≥ 1,8 und ≤ 2,6,
B) einer polymeren Polyether-Polyol-Komponente, welche ein Gemisch aus Poly(tetramethylenglykol)polyetherpolyolen enthält oder daraus besteht, wobei sich die Poly(tetramethylenglykol)polyetherpolyole in ihren zahlenmittleren Molekulargewichten unterscheiden,
C) einer aminofunktionellen Kettenverlängerer-Komponente mit mindestens 2 isocyanatreaktiven Aminogruppen, enthaltend wenigstens eine aminofunktionelle Verbindung C1), die keine ionischen oder ionogenen Gruppen aufweist und eine aminofunktionelle Verbindung C2), die ionische oder ionogene Gruppen aufweist,
D) gegebenenfalls weiteren hydrophilierenden Komponenten, die unterschiedlich sind von C2),
E) gegebenenfalls hydroxyfunktionellen Verbindungen mit einem Molekulargewicht von 62 bis 399 mol/g,
F) gegebenenfalls mindestens einem weiteren polymeren Polyol, welches unterschiedlich ist von B),
G) einer Verbindung, die genau eine isocyanatreaktive Gruppe aufweist, oder einer Verbindung, die mehr als eine isocyanatreaktive Gruppe aufweist, wobei nur eine der isocyanat reaktiven Gruppen unter den gewählten Umsetzungsbedingungen mit den in der Reaktionsmischung vorhandenen Isocyanatgruppen reagiert und
H) einer aliphatischen Polyisocyanat-Komponente mit einer mittleren Isocyanatfunktionalität von > 2,6 und ≤ 4,
wobei das molare Verhältnis von Komponente G) zu Komponente H) 5:1 bis 1:5 beträgt und
wobei die Komponenten B) und F) zusammen ≤ 30 Gew.-% an Komponente F), bezogen auf die Gesamtmasse der Komponenten B) und F) enthalten.

2. Produkt gemäß Anspruch 1, **dadurch gekennzeichnet, dass** die Komponente A) Isophorondiisocyanat und/oder Hexamethylendiisocyanat ist.

3. Produkt gemäß Anspruch 1 oder 2, **dadurch gekennzeichnet, dass** es sich bei der Komponente D) um nichtionisch hydrophilierende Komponenten handelt.

4. Produkt gemäß einem der Ansprüche 1 bis 3, **dadurch gekennzeichnet, dass** der Polyurethanharnstoff erhältlich ist indem isocyanatfunktionelle Polyurethanpräpolymere a) aus den Komponenten A), B) und gegebenenfalls D) und/oder C2), sowie gegebenenfalls den Verbindungen E) und/oder H) hergestellt werden und deren freie NCO-Gruppen anschließend ganz oder teilweise mit der aminofunktionellen Kettenverlängerer-Komponente C), sowie der Komponente G) und gegebenenfalls der Komponente D) und H) umgesetzt werden.

5. Produkt gemäß einem der Ansprüche 1 bis 4, **dadurch gekennzeichnet, dass** der Polyurethanharnstoff amorph ist und einen Tg ≤ - 25 °C, bestimmt mittels dynamischer Differenzkalorimetrie in Anlehnung an die DIN EN 61006, Verfahren A, aufweist.

6. Produkt gemäß einem der Ansprüche 1 bis 5, **dadurch gekennzeichnet, dass** das Substrat eine Höchstzugkraft von 100 bis 500 N, bestimmt gemäß DIN EN ISO 13934-1, aufweist.

7. Produkt gemäß einem der Ansprüche 1 bis 6, **dadurch gekennzeichnet, dass** das Produkt ein Pflaster, ein Verband, ein Tape oder eine Bandage oder zumindest ein Bestandteil dieser Endprodukte ist.

8. Verfahren zur Herstellung eines kontaktklebenden Produktes gemäß einem der Ansprüche 1 bis 7 umfassend die Schritte
I) Auftragen des Polyurethanharnstoffs auf das Substrat in Form einer wässrigen Polyurethanharnstoff-Dispersion und
II) thermische Trocknung des behandelten Substrates bei Temperaturen bei ≥ 20°C und ≤ 200°C.

9. Polyurethanharnstoff, welcher erhältlich ist, durch Umsetzung von wenigstens
A) einer aliphatischen Polyisocyanat-Komponente mit einer mittleren Isocyanatfunktionalität von ≥ 1,8 und ≤ 2,6,
B) einer polymeren Polyether-Polyol-Komponente, welche ein Gemisch aus Poly(tetramethylenglykol)polyetherpolyolen enthält oder daraus besteht, wobei sich die Poly(tetramethylenglykol)polyetherpolyole in ihren zahlenmittleren Molekulargewichten unterscheiden,
C) einer aminofunktionellen Kettenverlängerer-Komponente mit mindestens 2 isocyanatreaktiven Aminogruppen, enthaltend wenigstens eine aminofunktionelle Verbindung C1), die keine ionischen oder ionogenen Gruppen aufweist und eine aminofunktionelle Verbindung C2), die ionische oder ionogene Gruppen aufweist,
D) gegebenenfalls weiteren hydrophilierenden Komponenten, die unterschiedlich sind von C2),
E) gegebenenfalls hydroxyfunktionellen Verbindungen mit einem Molekulargewicht von 62 bis 399 mol/g,
F) gegebenenfalls weiteren polymeren Polyolen, welche unterschiedlich sind von B)
G) einer Verbindung, die genau eine isocyanatreaktive Gruppe aufweist, oder einer Verbindung, die mehr als eine isocyanatreaktive Gruppe aufweist, wobei nur eine der isocyanat reaktiven Gruppen unter den gewählten Umsetzungsbedingungen mit den in der Reaktionsmischung vorhandenen Isocyanatgruppen reagiert und
H) einer aliphatischen Polyisocyanat-Komponente mit einer mittleren Isocyanatfunktionalität von > 2,6 und ≤ 4,
wobei die Komponenten B) und F) zusammen ≤ 30 Gew.-% an Komponente F), bezogen auf die Gesamtmasse der Komponenten B) und F) enthalten und die Komponenten G) und H) in einem molaren Verhältnis von 5:1 bis 1:5 zueinander vorliegen.

10. Klebstoff umfassend einen Polyurethanharnstoff gemäß Anspruch 9.

11. Ein Objekt, hergestellt durch Verkleben von zwei oder mehr Substraten mittels eines Polyurethanharnstoffes gemäß Anspruch 9 oder eines Klebstoffes gemäß Anspruch 10.

12. Eine wässrige Dispersion enthaltend einen Polyurethanharnstoff gemäß Anspruch 9.

## Claims

1. Contact-adhesive product comprising a substrate and a polyurethaneurea obtainable by reacting at least
A) an aliphatic polyisocyanate component having an average isocyanate functionality of ≥ 1.8 and ≤ 2.6,
B) a polymeric polyetherpolyol component which contains or consists of a mixture of poly(tetramethylene glycol) polyetherpolyols, where the poly(tetramethylene glycol) polyetherpolyols differ in their number-average molecular weights,
C) an amino-functional chain extender component having at least 2 isocyanate-reactive amino groups, containing at least one amino-functional compound C1) that has no ionic or ionogenic groups and an amino-functional compound C2) that has ionic or ionogenic groups,
D) optionally further hydrophilizing components other than C2),
E) optionally hydroxy-functional compounds having a molecular weight of 62 to 399 mol/g,
F) optionally at least one further polymeric polyol other than B),
G) a compound having exactly one isocyanate-reactive group or a compound having more than one isocyanate-reactive group, where only one of the isocyanate-reactive groups reacts with the isocyanate groups present in the reaction mixture under the reaction conditions chosen, and
H) an aliphatic polyisocyanate component having an average isocyanate functionality of > 2.6 and ≤ 4,
where the molar ratio of component G) to component H) is 5:1 to 1:5 and
where components B) and F) together contain ≤ 30% by weight of component F), based on the total mass of components B) and F).

2. Product according to Claim 1, **characterized in that** component A) is isophorone diisocyanate and/or hexamethylene diisocyanate.

3. Product according to Claim 1 or 2, **characterized in that** component D) comprises nonionically hydrophilizing components.

4. Product according to any of Claims 1 to 3, **characterized in that** the polyurethaneurea is obtainable by preparing isocyanate-functional polyurethane prepolymers a) from components A), B) and optionally D) and/or C2), and optionally compounds E) and/or H), and the free NCO groups thereof are then wholly or partly reacted with the amino-functional chain extender component C), and also component G) and optionally components D) and H).

5. Product according to any of Claims 1 to 4, **characterized in that** the polyurethaneurea is amorphous and has a Tg ≤ -25°C, determined by means of dynamic differential calorimetry in accordance with DIN EN 61006, Method A.

6. Product according to any of Claims 1 to 5, **characterized in that** the substrate has a maximum tensile force of 100 to 500 N, determined to DIN EN ISO 13934-1.

7. Product according to any of Claims 1 to 6, **characterized in that** the product is a plaster, a dressing, a tape or a bandage or at least a constituent of these end products.

8. Process for producing a contact-adhesive product according to any of Claims 1 to 7, comprising the steps of
I) applying the polyurethaneurea to the substrate in the form of an aqueous polyurethaneurea dispersion and
II) thermally drying the treated substrate at temperatures ≥ 20°C and ≤ 200°C.

9. Polyurethaneurea obtainable by reacting at least
A) an aliphatic polyisocyanate component having an average isocyanate functionality of ≥ 1.8 and ≤ 2.6,
B) a polymeric polyetherpolyol component which contains or consists of a mixture of poly(tetramethylene glycol) polyetherpolyols, where the poly(tetramethylene glycol) polyetherpolyols differ in their number-average molecular weights,
C) an amino-functional chain extender component having at least 2 isocyanate-reactive amino groups, containing at least one amino-functional compound C1) that has no ionic or ionogenic groups and an amino-functional compound C2) that has ionic or ionogenic groups,
D) optionally further hydrophilizing components other than C2),
E) optionally hydroxy-functional compounds having a molecular weight of 62 to 399 mol/g,
F) optionally further polymeric polyols other than B),
G) a compound having exactly one isocyanate-reactive group or a compound having more than one isocyanate-reactive group, where only one of the isocyanate-reactive groups reacts with the isocyanate groups present in the reaction mixture under the reaction conditions chosen, and
H) an aliphatic polyisocyanate component having an average isocyanate functionality of > 2.6 and ≤ 4,
where components B) and F) together contain ≤ 30% by weight of component F), based on the total mass of components B) and F), and components G) and H) are present in a molar ratio of 5:1 to 1:5.

10. Adhesive comprising a polyurethaneurea according to Claim 9.

11. Object produced by bonding two or more substrates by means of a polyurethaneurea according to Claim 9 or an adhesive according to Claim 10.

12. Aqueous dispersion comprising a polyurethaneurea according to Claim 9.

## Revendications

1. Produit adhésif de contact, comprenant un substrat et une polyuréthane-urée qui peut être obtenue par mise en réaction d'au moins
A) un composant polyisocyanate aliphatique ayant une fonctionnalité isocyanate moyenne de ≥ 1,8 et ≤ 2,6,
B) un composant polyétherpolyol polymère qui contient ou consiste en un mélange de poly(tétraméthylèneglycol)-polyétherpolyols, les poly(tétraméthylèneglycol) polyétherpolyols se distinguant par leurs masses moléculaires moyennes en nombre,
C) un composant prolongateur de chaîne à fonction amino, comportant au moins 2 groupes amino réactifs avec des isocyanates, contenant au moins un composé à fonction amino C1) qui ne comporte pas de groupes ioniques ou ionogènes et un composé à fonction amino C2) qui comporte des groupes ioniques ou ionogènes,
D) éventuellement d'autres composants hydrophilisants qui sont différents de C2),
E) éventuellement des composés à fonction hydroxy ayant une masse moléculaire de 62 à 399 moles/g,
F) éventuellement au moins un autre polyol polymère qui est différent de B),
G) un composé qui comporte exactement un groupe réactif avec un isocyanate ou un composé qui présente plus d'un groupe réactif avec un isocyanate, un seul des groupes réactifs avec les isocyanates réagissant, dans les conditions réactionnelles choisies, avec les groupes isocyanate présents dans le mélange réactionnel et
H) un composant polyisocyanate aliphatique ayant une fonctionnalité isocyanate moyenne de > 2,6 et ≤ 4,
le rapport molaire du composant G) au composant H) valant de 5:1 à 1:5 et
les composants B) et F) contenant ensemble ≤ 30 % en poids de composant F), par rapport à la masse totale des composants B) et F).

2. Produit selon la revendication 1, **caractérisé en ce que** le composant A) est le diisocyanate d'isophorone et/ou le diisocyanate d'hexaméthylène.

3. Produit selon la revendication 1 ou 2, **caractérisé en ce que** pour ce qui est du composant D) il s'agit de composants hydrophilisants non ioniques.

4. Produit selon l'une quelconque des revendications 1 à 3, **caractérisé en ce que** la polyuréthane-urée peut être obtenue par préparation de prépolymères polyuréthane a) à fonction isocyanate à partir des composants A), B) et éventuellement D) et/ou C2), ainsi qu'éventuellement des composés E) et/ou H) et dont les groupes NCO libres sont ensuite en partie ou en totalité mis à réagir avec le composant prolongateur de chaîne C) à fonction amino, ainsi qu'avec le composant G) et éventuellement les composants D) et H).

5. Produit selon l'une quelconque des revendications 1 à 4, **caractérisé en ce que** la polyuréthane-urée est amorphe et présente une Tg ≤ -25 °C, déterminée par calorimétrie différentielle dynamique conformément à la norme DIN EN 61006, méthode A.

6. Produit selon l'une quelconque des revendications 1 à 5, **caractérisé en ce que** le substrat présente une résistance à la traction maximale de 100 à 500 N, déterminée selon DIN EN ISO 13934-1.

7. Produit selon l'une quelconque des revendications 1 à 6, **caractérisé en ce que** le produit est un pansement, une bande de contention, un ruban adhésif ou un bandage ou au moins une partie constituante de ces produits finals.

8. Procédé pour la production d'un produit adhésif de contact selon l'une quelconque des revendications 1 à 7, comprenant les étapes
I) application de la polyuréthane-urée sur le substrat, sous forme d'une dispersion aqueuse de polyuréthane-urée et
II) séchage thermique du substrat traité, à des températures de ≥ 20 °C et ≤ 200 °C.

9. Polyuréthane-urée qui peut être obtenue par mise en réaction d'au moins
A) un composant polyisocyanate aliphatique ayant une fonctionnalité isocyanate moyenne de ≥ 1,8 et ≤ 2,6,
B) un composant polyétherpolyol polymère qui contient ou consiste en un mélange de poly(tétraméthylèneglycol)polyétherpolyols, les poly(tétraméthylèneglycol)polyétherpolyols se distinguant par leurs masses moléculaires moyennes en nombre,
C) un composant prolongateur de chaîne à fonction amino, comportant au moins 2 groupes amino réactifs avec des isocyanates, contenant au moins un composé à fonction amino C1) qui ne comporte pas de groupes ioniques ou ionogènes et un composé à fonction amino C2) qui comporte des groupes ioniques ou ionogènes,
D) éventuellement d'autres composants hydrophilisants qui sont différents de C2),
E) éventuellement des composés à fonction hydroxy ayant une masse moléculaire de 62 à 399 moles/g,
F) éventuellement d'autres polyols polymères qui sont différents de B),
G) un composé qui comporte exactement un groupe réactif avec un isocyanate ou un composé qui présente plus d'un groupe réactif avec un isocyanate, un seul des groupes réactifs avec les isocyanates réagissant, dans les conditions réactionnelles choisies, avec les groupes isocyanate présents dans le mélange réactionnel et
H) un composant polyisocyanate aliphatique ayant une fonctionnalité isocyanate moyenne de > 2,6 et ≤ 4,
les composants B) et F) contenant ensemble ≤ 30 % en poids de composant F), par rapport à la masse totale des composants B) et F) et les composants G) et H) étant présents l'un par rapport à l'autre en un rapport molaire de 5:1 à 1:5.

10. Adhésif comprenant une polyuréthane-urée selon la revendication 9.

11. Objet, fabriqué par collage de deux ou plus de deux substrats ai moyen d'une polyuréthane-urée selon la revendication 9 ou d'un adhésif selon la revendication 10.

12. Dispersion aqueuse contenant une polyuréthane-urée selon la revendication 9.
